(19) Europäisches Patentamt
European Patent Office
Office européen des brevets

(11) Publication number: **0 355 819 B1**

(12) **EUROPEAN PATENT SPECIFICATION**

(45) Date of publication of patent specification: **05.01.94**

(51) Int. Cl.[5]: **C07C 275/42**, C07C 275/30, C07C 311/47, C07C 335/22, C07D 213/40, C07D 213/81, C07D 213/84, A23L 1/236

(21) Application number: **89115587.1**

(22) Date of filing: **23.08.89**

The file contains technical information submitted after the application was filed and not included in this specification

(54) **Substituted aryl ureas as high potency sweeteners.**

(30) Priority: **23.08.88 US 235396**
**21.08.89 US 395242**

(43) Date of publication of application:
**28.02.90 Bulletin 90/09**

(45) Publication of the grant of the patent:
**05.01.94 Bulletin 94/01**

(84) Designated Contracting States:
**AT BE CH DE FR GB GR IT LI LU NL SE**

(56) References cited:
**DE-A- 3 236 626**
**GB-A- 707 590**
**GB-A- 2 087 870**
**US-A- 3 121 111**
**US-A- 4 645 678**

**DEVELOPMENTS IN SWEETENERS-1, C. A.M. HOUGH et al, Applied Science Publishers LTD., London, GB, pages 159, 160**

(73) Proprietor: **THE NUTRASWEET COMPANY (a Delaware corporation)**
**1751 Lake Cook Road**
**Deerfield Illinois 60015(US)**

(72) Inventor: **Madigan, Darold L.**
**908 Wisconsin Lane**
**Elk Grove Village, IL 60007(US)**
Inventor: **Muller, George W.**
**1915 Smith Road**
**Northbrook, IL 60062(US)**
Inventor: **Walters, D. Eric**
**643 N. Emerald Avenue**
**Mundelein, IL. 60060(US)**
Inventor: **Culberson, John C.**
**229 S. Salem Drive**
**Schaumburg, IL 60193(US)**
Inventor: **Dubois, Grant E.**
**37, Ouail Drive**
**Lake Forest, IL. 60045(US)**
Inventor: **Carter, Jeffrey S.**
**708 Stephan Drive**
**Palatine, IL. 60067(US)**
Inventor: **Nagarajan, Srivivasan**
**700 W. Rand Road**
**Arlington Heights, IL. 60004(US)**

**EP 0 355 819 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Inventor: **Klix, Russell C.**
**4232 Bloomington Ave., App. 204**
**Arlington-Heights, IL 60004(US)**
Inventor: **Ager, David J.**
**4700 Arbor Drive, App.115**
**Rolling Meadows, IL. 60008(US)**
Inventor: **Klade, Carrie A.**
**P.O.Box 1539**
**King of Prussia, Pennsylvania**
**19406-0939(US)**

(74) Representative: **Beil, Hans Chr., Dr. et al**
**BEIL, WOLFF & BEIL,**
**Rechtsanwälte,**
**Postfach 80 01 40**
**D-65901 Frankfurt (DE)**

## Description

This application is a continuation in part of U.S. Serial No. 07/235,396, which is incorporated herein by reference. The present invention relates to substituted aryl ureas and thioureas which are useful as sweetening agents. Additionally, the present invention relates to methods of preparing the novel compounds, as well as sweetening compositions and food products containing ureas and thioureas as sweeteners.

Certain urea and thiourea derivatives are known in the art as sweeteners. The commonly known sweetener, suosan, for example, has the structure

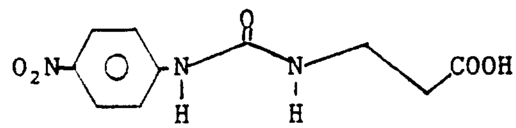

Suosan was reported by Petersen and Muller (Chem. Ber. 1948, 81, 31 and Angew, Chem. 1948, 60A, 58). Other examples of urea and thiourea compounds are found in Z. Lebensm Unters. Forsch. 1982, 175, 266; Japanese Patent 61-260052; Rec. Trav. Chim. 1883, 2, 121; Rec. Trav. Chim. 1884, 3, 223; and J. American Chemical Society 1926, 48, 1069; Naturwissenaschaften 1980, 67, 193; and Naturwissenschaften 1981, 68, 143; and U.S. Patent No. 4,645,678 to Nofre et al.

## SUMMARY OF THE INVENTION

In accordance with the present invention, substituted ureas are useful as sweetening agents. (For purposes of this application, the term "urea" includes inventive compounds which are ureas and thioureas.) The present ureas may be added to food products in amounts sufficient to sweeten food to a desired sweetness level.

The inventive ureas may be prepared by reacting an isocyanate or isothiocyanate with an amine or aniline. A wide variety of ureas may be manufactured by this method.

Particularly desirable urea compounds include: N-(4-carbamoylphenyl)-N'-[3-(3-phenylpropionic acid)] urea,
N-(4-cyanophenyl)-N'-[3-(3-phenylpropionic acid)] urea,
N-(4-cyanophenyl)-N'-[3-(3-(3-pyridyl)propionic acid)] urea,
N-(4-ethoxycarbonylphenyl)-N'-[3-(3-phenylpropionic acid)] urea,
N-(4-ethoxycarbonylphenyl)-N'-[3-(3-(3-pyridyl)propionic acid)] urea,
N-(4-nitrophenyl)-N'-[3-(3-phenylpropionic acid)] urea,
N-(4-nitrophenyl)-N'-[3-(3-(3-pyridyl)propionic acid)] urea, and
N-(4-formylphenyl)-N'-[3-(3-(3-pyridyl)propionic acid)] urea.
N-(4-carbamoylphenyl)-N'-[3-(3-(3-pyridyl)propionic acid)]urea.
N-[5-(2-cyanopyridyl)]-N'-[3-(3-phenylpropionic acid)]urea
N-[5-(2-cyanopyridyl)]-N'-[3-(3-(3-pyridyl)propionic acid)]urea
N-[5-(2-carbamoylpyridyl)]-N'-[3-(3-phenylpropionic acid)]urea
N-[5-(2-carbamoylpyridyl)]-N'-[3-(3-(3-pyridyl)propionic acid)]urea
N-[5-(2-formylpyridyl)]-N'-[3-(3-phenylpropionic acid)]urea
N-[5-(2-formylpyridyl)]-N'-[3-(3-(3-pyridyl)propionic acid)]urea

## Detailed Description of the Preferred Embodiment

The present substituted ureas are represented by the following formula:

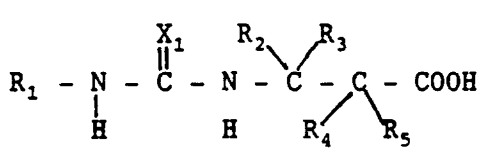

(I)

wherein $X_1$ is S or O, wherein $R_1$ is an optionally substituted phenyl group, where the phenyl corresponds to:

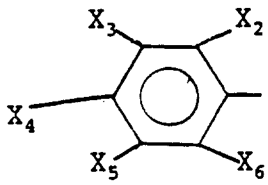

wherein $X_2$, $X_3$, $X_4$, $X_5$ and $X_5$ are the same or different and are selected from the group consisting of:

H,
$CF_3$,
$CF_2CF_3$,
$CH_2CF_3$,
$C_1$-$C_4$ alkyl,
$CH=NOCH_3$,
$CH=NOH$,
CHO,
$CH_2OCH_3$,
$CH_2OH$,
CN,
$COCF_3$,
$COC_1$-$C_3$ alkyl,
$CONH_2$,
$CONHC_1$-$C_3$ alkyl,
$CON(C_1$-$C_3$ alkyl$)_2$,
$COOC_1$-$C_3$ alkyl,
COOH,
$NH_2$,
$NHC_1$-$C_3$ alkyl,
$N(C_1$-$C_3$ alkyl$)_2$,
Br,
Cl, with the proviso that $X_3$ and $X_5$ are not both Cl,
F,
I,
NHCHO,
$NHCOCH_3$,
$NHCONH_2$,
$NHSO_2CH_3$,
$C_1$-$C_3$ alkyl COOH,
$NO_2$,
$OC_1$-$C_3$ alkyl, with the proviso that $X_4$ is not $OCH_2CH_3$ $OCOCH_3$,
OH,
$SC_1$-$C_3$ alkyl,
$SOC_1$-$C_3$ alkyl,
$SO_2C_1$-$C_3$ alkyl,
$SO_2NH_2$,
$SO_2NHC_1$-$C_3$ alkyl,
$SO_2N(C_1$-$C_3$ alkyl$)_2$,
$SO_3H$,
and where substituents at any two of $X_2$, $X_3$, $X_4$, $X_5$ or $X_5$ form a fused ring,

or wherein $R_1$ is selected from the group consisting of optionally substituted pyridyl, optionally substituted pyrimidyl, 2-indanyl, or 6-indazolyl, and wherein $R_3$, $R_4$ and $R_5$ are hydrogen and wherein $R_2$ is phenyl optionally substituted by one or two alkoxy groups or a nitro group, pyridyl or 2-phenylethyl.

Suitable heterocyclic moieties for $R_1$ include optionally substituted pyridines, thiazoles, indoles, naphthyridines, cinnolines, pteridines, thiophenes, benzothiophenes, naphthothiophenes, thianthrenes, furans,

pyrans, isobenzofurans, chromenes, xanthenes, phenoxanthins, pyrroles, isoindoles, indolizines, pyridazines, pyrimidines, pyrazines, pyrazoles, imidazoles, pyrroles, indazoles, purines, quinolizines, isoquinolines, quinolines, phthalazines, quinoxalines, quinazolines, carbazoles, carbolines, phenanthridines, acridines, pyrimidines, phenanthrolines, phenazines, phenarsazines, isothiazoles, phenothiazines, isoxazoles, tetrazoles, triazoles, furazans and heterocyclics of the following formulas:

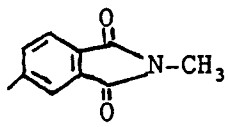 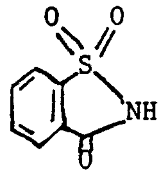 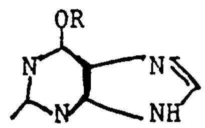

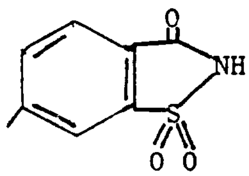 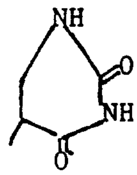

wherein R is H or $C_1$-$C_6$ alkyl. The heterocyclic moieties may be optionally substituted with one or more substituents, such as, for example, $C_1$-$C_6$ alkyl, halogen, $NO_2$, CN, trihalomethyl, carbamoyl, formyl, dihalomethyl, hydroxyl or hydroxyalkyl.

Preferred $R_2$ substituents include

H,

pyridyl and substituted pyridyl

phenyl and substituted phenyl.

Specifically preferred $R_2$ substituents include S-phenylethyl and pyridyl.

Preferably, the inventive urea is one where $R_1$ is

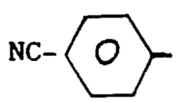 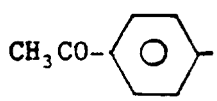 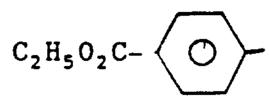

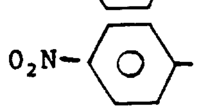 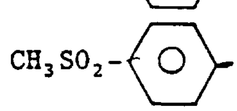 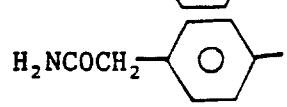

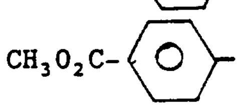 2-indanyl 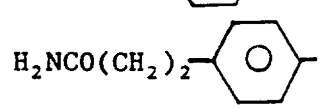

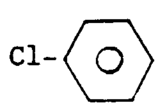 6-indazolyl 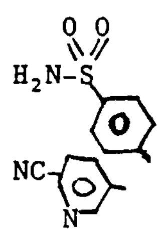

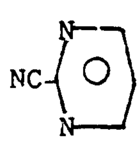 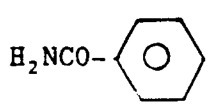

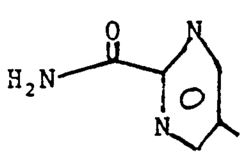 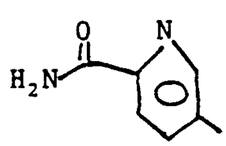

$R_2$ is phenyl, 3-pyridyl, 2-pyridyl, 4-pyridyl, 4-methoxyphenyl.
$R_3$, $R_4$, and $R_5$ are H and
$X_1$ is O.

There are two isomeric forms (R) and (S) of some preferred compound. The form having more sweetening potency is believed to be the (S) isomer, and is preferred for purposes of this invention.

Particularly preferred compounds include those wherein
$R_1$ is

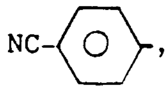

$R_2$ is 3-pyridyl, $R_3$, $R_4$, and $R_5$ are H, and $X_1$ is O,
$R_1$ is

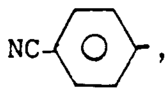

$R_2$ is phenyl, $R_3$, $R_4$, and $R_5$ are H and $X_1$ is O,
$R_1$ is

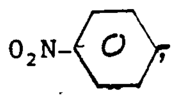

$R_2$ is 3-pyridyl, $R_3$, $R_4$, and $R_5$ and H and $X_1$ is O,
$R_1$ is

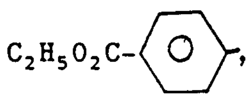

$R_2$ is 3-pyridyl, $R_3$, $R_4$, and $R_5$ are H and $X_1$ is O,
$R_1$ is

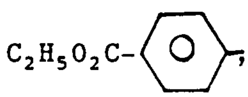

$R_2$ is phenyl, $R_3$, $R_4$, and $R_5$ are H and $X_1$ is O,
$R_1$ is

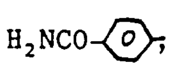

$R_2$ is phenyl, $R_3$, $R_4$, and $R_5$ and H and $X_1$ is O
and $R_1$ is

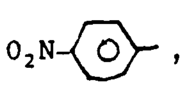

$R_2$ is phenyl, $R_3$, $R_4$, and $R_5$ are H and $X_1$ is O,
$R_1$ is

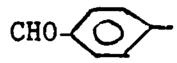

,

$R_2$ is 3-pyridyl, $R_3$, $R_4$, and $R_5$ are H and $X_1$, is O,
$R_1$ is

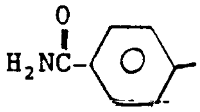

,

$R_2$ is 3-pyridyl, $R_3$, $R_4$, and $R_5$ are H and $X_1$ is O,
$R_1$ is

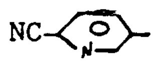

,

$R_2$ is phenyl, $R_3$, $R_4$, and $R_5$ are H and $X_1$ is O,
$R_1$ is

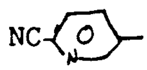

,

$R_2$ is 3-pyridyl, $R_3$, $R_4$ and $R_5$ are H and $X_1$ is O
$R_1$ is

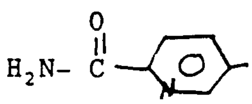

,

$R_2$ is phenyl, $R_3$, $R_4$ and $R_5$ are H and $X_1$ is O
$R_1$ is

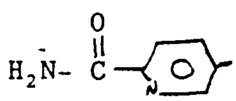

,

$R_2$ is 3-pyridyl, $R_3$, $R_4$, and $R_5$ are H and $X_1$ is O
$R_1$ is

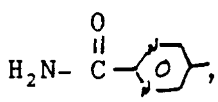

,

$R_2$ is phenyl, $R_3$, $R_4$, and $R_5$ are H and $X_1$ is O
$R_1$ is

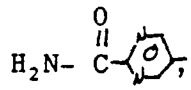

$R_2$ is 3-pyridyl, $R_3$, $R_4$, and $R_5$ are H and $X_1$ is O

$R_1$ is

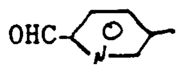 ,

$R_2$ is phenyl, $R_3$, $R_4$, and $R_5$ are H and $X_1$ is O

$R_1$ is

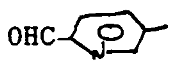 ,

$R_2$ is 3-pyridyl, $R_3$, $R_4$, and $R_5$ are H and $X_1$ is O

$R_1$ is

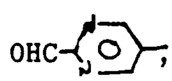

$R_2$ is phenyl, $R_3$, $R_4$, and $R_5$ are H and $X_1$ is O

$R_1$ is

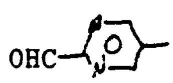 ,

$R_2$ is 3-pyridyl, $R_3$, $R_4$, and $R_5$ are H and $X_1$ is O.

The present ureas also include physiologically acceptable salts of the compounds described above. The ureas also may have two asymmetrical carbon atoms, i.e., optically active sites as asterisked in the following structure:

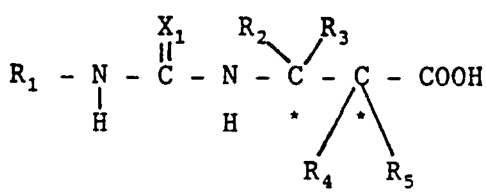

These ureas exist in (R) and (S) enantiomeric forms if there is one optically active site. If both sites are optically active, there are four possible diastereomic forms: (R)(R), (R)(S), (S)(R), and (S)(S).

The present invention also relates to edible products containing the present urea compounds as sweetening agents either alone or in combination with other sweeteners. Also provided by the present invention is a process for sweetening edible products such as foods, beverages, chewing gums, confections, pharmaceuticals, veterinary preparations and the like.

The present invention further contemplates compositions of the present ureas in combination with other sweetening agents and/or physiologically acceptable carriers which may be bulking agents. Suitable carriers include water, polymeric dextrose such as polydextrose, starch and modified starches, maltodextrins, cellulose, methylcellulose, maltitol, cellobiitol, carboxymethylcellulose, hydroxypropylcellulose, hemicelluloses microcrystalline cellulose, other cellulose derivatives, sodium alginate, pectins and other gums,

lactose, maltose, glucose, leucine, glycerol, mannitol, sorbitol, sodium bicarbonate and phosphoric, citric, tartaric, fumaric, benzoic, sorbic, propionic acids and their sodium, potassium and calcium salts and mixtures of all of the above.

Suitable sweetening agents which may be used in combination with the present ureas can be sugars or high potency sweeteners such as sucrose, corn syrups, fructose, high fructose corn syrup, aspartame, alitame, neohesperidin dihydrochalcone, hydrogenated isomaltulose (Palatinite), stevioside type sweeteners, L-sugars, glycyrrhizin, xylitol, lactitol, neosugar, acesulfam-K, saccharin (sodium, potassium or calcium salt), cyclamic acid (sodium, potassium or calcium salt), sucralose, monellin and thaumatin and mixtures thereof.

The present invention also relates to a novel method of preparing the inventive urea compounds. An isocyanate of the formula

$R_1NCX_1$

with $R_1$ and $X_1$ chosen as desired from the substituents earlier disclosed is reacted with a substituted beta-amino acid, such as a beta-alanine of the formula

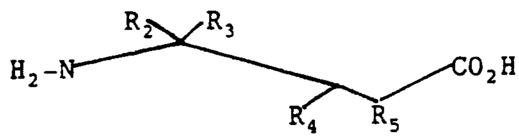

with $R_2$, $R_3$, $R_4$, and $R_5$ chosen as desired from the substituents earlier disclosed. The ester of the β amino acids may also be used. The substituted beta-amino acid may be prepared by the methods disclosed in:

U.S. Patent 4,127,570 to Fosker
Journal of the Chemical Society (1936), V.59, p.299
Journal of the Chemical Society (1929), V.51, P.41
Liebigs Ann. Chemistry (1981), V.12, p.2258
Synthetic Communication (1981), V.11, p.95
Synthesis (1982), p. 967
Chem. Pharm. Bull. (1978), 26, 260-263
each of which is incorporated herein by reference.

The isocyanate and substituted beta-amino acid may be reacted in the presence or absence of a base. The reaction is preferably carried out in the presence of a solvent such as acetonitrile, a mixture of acetonitrile and water, methanol, acetone, or a mixture of ethyl acetate and water.

Anilines may also be reacted with isocyanates or isothiocyanates of a substituted β-amino acid ester followed by ester hydrolysis.

In some of the desired compounds, it is preferable to isolate one of two enantiomeric forms. An aldehyde and a chiral amine are reacted to produce a Schiff base. The Schiff base is reacted with a methyl haloacetate in THF with a metal such as zinc to produce a diastereomeric mixture of a β-lactam. The desired diastereomer is separated after the β-lactam is hydrolyzed and esterified to produce an ester of a first β-amino acid. After hydrogenolysis, the desired stereoisomer of a second β-amino acid is obtained.

For some applications, esterification is not necessary. In these applications, the desired diastereomer of the β-lactam is isolated and then hydrolyzed to produce a diastereomeric mixture of a first β-amino acid. The first β-amino acid is then hydrogenolyzed to produce the desired stereoisomer of a second β-amino acid.

The present invention also relates to a method of sweetening foods or comestible products. In such uses, the present ureas are added to any consumable product in which it is desired to have a sweet taste. The inventive urea compounds are added to such products in amounts effective to impact the desired level of sweetness. The optimum amount of the urea sweetener agent will vary depending on a variety of factors, including the sweetness potency of a particular urea sweetening agent, storage and use conditions of the product, the particular components of the product, the flavor profile of the comestible product, and the level of sweetness desired. One skilled in the art can readily determine the optimum amount of sweetening agent to be employed in a particular formulation of a food product by conducting routine sweetness (sensory) experiments. Usually, the present sweetening agents are added to the comestible products in amounts of from about 0.00001 to about 0.1 percent by weight of the comestible product, advantageously from about

0.00005 to about 0.05 weight percent and preferably from about 0.001 to about 0.02 weight percent. Concentrates, of course, will contain higher percentages of sweetening agent(s), and are diluted for end use purposes.

Suitable products which are sweetened by the present sweetening agents include any products for which a sweet flavor component is desired such as food products (for human or animal consumption), beverages (alcoholic, soft drinks, juices, carbonated beverages), confectionary products (candies, chewing gum, baked goods, pastries, breads, etc.), hygiene products, cosmetics, pharmaceutical products and veterinary products. In sweetening gum, the present ureas can be added in amounts in excess of a sucrose equivalent normally found in gum. This excess amount of urea sweetener may provide a longer sweet taste due to its lower solubility compared to sucrose and enhancement of flavor (flavor enhancer).

The present ureas can be added in pure form to foods to impart a sweet flavor. However, because of the high sweetness potency of the present sweetening agents, they are typically admixed with a carrier or bulking agent. Suitable carriers or bulking agents include water, polymeric dextrose such as Polydextrose, starch and modified starches, maltodextrins, cellulose, hemicellulose, methylcellulose, carboxymethylcellulose, cellobiitol, hydroxypropylcellulose, hemicelluloses microcrystalline cellulose, cellulose derivatives, sodium alginate, pectins and other gums, lactose, maltose, maltitol, glucose, leucine, glycerol, mannitol, sorbitol, sodium bicarbonate and phosphoric, citric, tartaric, fumaric, benzoic, sorbic and propionic acids and their sodium, potassium and calcium salts and mixtures of all of the above.

The present ureas can be employed alone as the sole sweetening agent in a comestible product. Mixtures of more than one of the inventive ureas can also be employed. Additionally, the ureas can be used in combination with other sweetening agents such as sugars (such as fructose and sucrose), corn syrups, high potency sweeteners such as aspartame and alitame, and other sweeteners such as glycyrrhizin, aminoacyl sugars, xylitol, sorbitol, mannitol, acesulfam K, thaumatin, monellin, cyclamates, saccharin, neohesperidin dihydrochalcone, hydrogenated isomaltulose, (Palatinit), stevioside type sweeteners, lactitol, neosugar, L-sugars, sucralose, and mixtures thereof.

The compounds synthesized were tasted as aqueous solutions at 1 mg/ml and 10 fold dilutions thereof and compared in taste quality and intensity to a sucrose standard solution. All compounds were found to be sweet.

The following examples illustrate the practice of the present invention, but should not be construed as limiting its scope.

## EXAMPLES

### EXAMPLE 1

Preparation of N-(4-Ethoxycarbonylphenyl)-N'-[3-(3-phenylpropionic acid)]urea.

To a stirred solution of 4-ethoxycarbonylphenyl isocyanate (2.16 g, 11.3 mmol) in 35 ml of acetonitrile was added a solution of 3-amino-3-phenylpropionic acid (1.90 g, 11.5 mmol) and sodium hydroxide (0.458 g, 11.5 mmol) in a mixture of 6 ml of water and 6 mL of acetonitrile. The reaction mixture was stirred for 16 hours, then concentrated. The residue was diluted with water (50 ml) and extracted with methylene chloride (25 mL) and ethyl acetate (25 ml). The aqueous layer was acidified with 11.5 mL of 1 N HC1 and stirred for 30 minutes. The resulting slurry was filtered and the solid was washed with copious amounts of water. The solid was dried in vacuo to afford 3.61 g (90%) of the urea as white powder. PMR (dmso-$D_6$) $\delta$ 12.3 (s, 1 H), 9.03 (s, 1 H), 7.82, 7.50 (abq, 4H), 7.45-7.2 (m, 5 H), 6.96 (d, 1H, J = 8.4 Hz), 5.14 (overlapping dt, 1H), 4.24 (q, 2 H, J = 7 Hz), 2.78 (m, 2 H), 1.28 (t, 3 H, J = Hz). CMR (dmso-$D_6$) $\delta$ 172.0, 165.5, 153.9, 144.9, 142.6, 130.3, 128.3, 127.0, 126.3, 122.1, 116.7, 60.2, 50.0, 40.9, 14.2 IR(KBr)$cm^{-1}$ 3400, 3340, 3200, 2980, 1710, 1650, 1595, 1553, 1512, 1409. Anal. calcd. for $C_{19}N_{20}N_2O_5$-0.17 $H_2O$: C, 63.49; H, 5.70; N, 7.79. Found: C, 63.47: H, 5.68: N, 7.63.

### EXAMPLE 2

Preparation of N-(4-Acetylphenyl)-N'-[3-(3-phenylpropionic acid)]urea.

To a stirred solution of 4-acetylphenyl isocyanate (1.87 g, 11.6 mmol) in 35 mL of acetonitrile was added a solution of 3-amino-3-phenylpropionic acid (1.95 g, 11.8 mmol) and sodium hydroxide (0.472 g, 11.8 mmol) in a mixture of 6 ml of water and 6 ml of acetonitrile. Solid formed in the reaction material immediately. The reaction mixture was stirred for 17 hours, then concentrated. The residue was diluted with

water (75 ml) and extracted with ethyl acetate (2 x 25 mL ea.) The aqueous layer was concentrated to remove traces of ethyl acetate. The aqueous layer was then acidified with 14 ml of 1 N HC1 and the product gummed out. The resulting suspension was stirred and the gum solidified. The slurry was filtered and the solid was washed with copious amounts of water. The solid was dried in vacuo to afford 3.30 g (87%) of the urea as tan powder. The crude product was recrystallized from acetonitrile to afford 1.67 g (44%) of the urea. PMR (dmso-$D_6$) δ 12.3 (s, 1 H), 9.01 (s, 1 H), 7.81 (d, 2 H, J = 8.8 Hz), 7.47 (d, 2H, J = 8.8 Hz), 7.4-7.15 (m, 5 H), 6.95 (d, 1H, J = 8.4 Hz), 5.11 (apparent q, 1 H), 2.85-2.6 (m, 2 H), 2.45 (s, 3 H). CMR (dmso-$D_6$) δ 196.2, 172.0, 153.9, 144.9, 142.6, 129.6, 128.3, 127.0, 126.3, 116.7, 49.9, 40.9, 26.3.

EXAMPLE 3

Preparation of N-(4-Bromophenyl)-N'-[3-(3-phenylpropionic acid)]urea.

To a stirred solution of 4-bromophenyl isocyanate (2.69 g, 13.6 mmol) in 35 mL of acetonitrile was added a solution of 3-amino-3-phenylpropionic acid (2.29 g, 13.9 mmol) and sodium hydroxide (0.555 g, 13.9 mmol) in a mixture of 6 ml of water and 6 mL of acetonitrile. The reaction mixture was stirred for 24 hours, then concentrated. The residue was diluted with water (75 ml) and extracted with ethyl acetate (2 x 50 ml). The aqueous layer was concentrated to remove traces of ethyl acetate and then acidified with 20 mL of 1 N HC1. The resulting thick slurry was diluted with water and filtered. The solid was washed with copious amounts of water and dried in vacuo to afford 3.61 g (90%) of the urea as white powder. PMR (dmso-$D_6$) δ 12.3 (bs, 1 H), 8.73 (s, 1 H), 7.45-7.2 (m, 9H), 6.84 (d, 1H, J = 8.4 Hz), 5.11 (apparent q, 1 H), 2.85-2.65 (m, 2 H). CMR (dmso-$D_6$) δ 172.0, 154.1, 142.7, 139.7, 131.4, 128.3, 126.9, 126.3, 119.5, 112.4, 49.9, 40.9.

EXAMPLE 4

Preparation of N-(4-cyanophenyl)-N'-[3-(3-phenylpropionic acid)]urea

To a solution of 1.652 g (11.5 mmol) of 4-cyanophenyl isocyanate in 50 mL acetonitrile was added 1.893 g (11.5 mmol) of 3-amino-3-phenylpropionic acid slurried in 50 ml acetonitrile. After 1 hour at room temperature the reaction mixture was heated to reflux where, after the addition of an additional 50 ml of acetonitrile, a clear solution formed. The reaction mixture was cooled with stirring overnight. The solids were filtered off and dried at 40° C/1 mm Hg to a constant weight of 3.01 g (84.6%) of the desired urea, m.p. 190-192° C IR (KBr) 3380, 3320, 2230, 1680, 1600, 1540, 1320, 1240 $cm^{-1}$. $^1H$ NMR ($Me_2SO$-$d_6$, 300MHZ) δ 2.6-2.7 (d,2H), 4.9-5.1 (m,1H), 6.9 (d,1H), 7.0-7.6 (m, 9H), 9.0 (s,1H); $^{13}C$ NMR ($Me_2SO$-$d_6$, 75.5MHZ) δ 172.8, 154.6, 145.6, 143.3, 134.0, 127.8, 127.1, 120.2, 118.3, 103.4, 50.8, 41.6. Anal. Calcd for $C_{17}H_{15}N_3O_3$: C, 66.01; H, 4.89; N, 13.59. Found: C, 66.15; H, 4.92; N, 13.92.

EXAMPLE 5

Preparation of N-(4-Cyanophenyl)-N'-[3-(3-(3-pyridyl)propionic acid)]urea Sodium salt

To a solution of 1.66 g (10 mmol) of 3-amino-3-(3-pyridyl) propionic acid, 0.4 g of NaOH, and 50 ml $H_2O$ was added 2.88 g (20 mmol) of 4-cyanophenyl isocyanate in 50 ml ethyl acetate. The reaction mixture was stirred overnight at room temperature. The two phase mixture was filtered to remove traces of impurities and the aqueous phase was twice extracted with ethyl acetate. The water was removed at reduced pressure to produce a gummy mass. TLC and $^1H$ NMR indicated the material to be a mixture of desired urea and starting beta-amino acid. The desired urea was isolated by reverse phase chromatography using acetonitile/water as the mobile phase. IR (KBr) 3400, 2230, 1700, 1600, 1560, 1400 $cm^{-1}$. $_1H$ NMR ($Me_2SO$-$d_6$, 300MHz) δ 2.5 (d,2H), 5.1 (s,1H), 7.3 (m,1H), 7.5 (d,2H), 7.6 (d,2H), 7.65 (s,1H), 8.35 (d,1H), 8.55 (s,1H), 9.3 (d,1H); $^{13}C$ NMR ($Me_2SO$-$d_6$, 75.5 MHz) δ 174.8, 154.7, 147.8, 147.0, 146.1, 140.7, 133.5, 132.6, 123.0, 119.6, 117.2, 101.0, 49.7, 45.0.

EXAMPLE 6

Preparation of N-(4-Nitrophenyl)-N'-[3-(3-phenylpropionic acid)]urea

To a slurry of 1.652 g (10 mmol) of 3-amino-3-phenylpropionic acid in 50 ml acetone was added 1.641 g (10 mmol) of 4-nitrophenyl isocyanate dissolved in 5 ml acetone. After 4 hours of stirring at room temperature, a trace of insoluble impurities was removed by filtration. After removal of the solvent a bright yellow solid was isolated in a quantitative yield. The crude product was purified on a silica column using a chloroform:methanol:acetic acid solvent. IR (KBr) 3400, 1700, 1560, 1500, 1350 $cm^{-1}$. $^1H$ NMR ($Me_2SO-d_6$, 300MHz) δ 2.75 (bs,2H), 5.2 (d,1H), 7.2-7.4 (m,5H), 7.65 (d,2H), 7.85 (m,1H), 8.1 (d,2H), 10.1 (s,1H); $^{13}C$ NMR ($Me_2SO-d_6$, 75.5 MHz) δ 153.9, 147.4, 143.3, 140.2, 128.1, 126.6, 126.3, 124.9, 116.7, 50.5. Anal. Calcd for $C_{16}H_{15}N_3O_5(2H_2O)$: C, 52.59; H, 5.24: N, 11.50. Found: C, 52.14; H, 4.70; N, 11.56.

EXAMPLE 7

Preparation of N-4-Carbamoylphenyl-N'-(3-(3-phenylpropionicacid) urea

Methyl 3-isocyanato-3-phenylpropionate was first prepared.

The reaction assembly is as follows: a 100 mL three-neck round bottom flask was fitted with a thermometer, reflux condenser, and gas inlet bubble tube. The condenser was connected to a trap and then to an aqueous NaOH bath (phosgene scrubber). The gas inlet line consisted of a T-tube with nitrogen and phosgene inlets at two of the openings. The exit led through a trap and into the gas bubble tube.

The apparatus was purged with nitrogen, toluene (20 mL) was added and the solution chilled in an ice-salt bath to 0 °C. Gaseous phosgene (10 mL, 14 g, 140 mmol; actual measurement based on volume increase of the toluene solution) was added and a slow addition of phosgene was continued throughout the remainder of the reaction. Methyl 3-phenyl-3-aminopropionate was added portionwise over 2 min. to the phosgene solution. The reaction mixture was stirred at 0 °C for 15 min, allowed to warm to room temperature over 30 min, and then carefully heated and held at 110 °C for 4 hours (slow phosgene addition was continued). The resulting clear solution was allowed to cool to room temperature, purged with nitrogen overnight and then concentrated (asp vacuum) yielding an oil. Vacuum distillation using a Kugelrohr apparatus (70 °C, 1 mm) afforded the pure isocyanate (8.95 g, 94 %): $^1H$ NMR ($CDCl_3$) δ 7.42 (m, 5 H), 5.12 (q, J = 4.7 Hz, 1 H), 3.71 (s, 3 H), 2.79 (m, 2 H); IR (thin film) $cm^{-1}$ 2251, 1745, 1438, 1269, 1199, 1170, 987, 760, 700. Anal. Calcd for $C_{11}H_{11}N_1O_3$: C, 64.38; H, 5.40; N, 6.83. Found: C, 64.52; H, 5.55; N, 6.81.

N-(4-Carbamoylphenyl)-N'-[(3-(methyl 3-phenylpropionate)] was then prepared by the following procedure.

To a solution of methyl 3-isocyanato-3-phenylpropionate (1.97 g, 9.59 mmol) in $CH_3CN$ (35 mL) was added 4-aminobenzamide (1.31 g, 9.59 mmol) with stirring at room temperature. The resulting clear solution was allowed to stand for 3 weeks during which time a white precipitate formed. Vacuum filtration yielded the desired urea (3.06 g, 94 %) as a white solid; mp 198-200 °C; $^1H$ NMR ($DMSOd_6$) δ 8.78 (s, 1 H), 7.71 (d, J = 9.3 Hz, 2 H), 7.37 (d, J = 9.3 Hz, 2 H), 7.36-7.18 ( m, 5 H), 7.10 (s, 1 H), 6.88 (d, J = 7.8 Hz, 1 H), 5.12 (q, J = 7.8 Hz, 1 H), 3.54 (s, 3 H), 2.82 (m, 2 H); IR (KBr) $cm^{-1}$ 3354, 1730, 1669, 1659, 1528, 701. Anal. Calcd for $C_{18}H_{19}N_3O_4$: C, 63.33; H, 5.61; N, 12.31. Found: C, 63.29; H, 5.82; N, 12.43.

LiOH (0.31 g, 7.3 mmol) in $H_2O$ (5 mL) was added via syringe pump over 4 hr to a solution of N-(4-carbamoylphenyl)-N'-[3-(methyl 3-phenylpropionate)] (2.50 g, 7.32 mmol) in $CH_3OH/H_2O$ (2:1, 75 mL). The resulting suspension was stirred for 36 hr and filtered. The aqueous filtrate was washed with methylene chloride (3 X 25 mL) and then acidified to pH 3 with 1 N HCl, yielding the desired acid, N-(4-carbamoylphenyl)-N'-[3-(3-phenylpropionic acid)]urea (1.75 g, 73 %) as a white solid: mp 201-212 °C with decomp; $^1H$ NMR ($CD_3OD$) δ 8.82 (s, 1 H), 7.76 (s) and 7.71 (d, J = 8.6), (3 H), 7.37 (d, J = 8.6 Hz, 2 H), 7.34-7.17 (m, 5 H), 7.09 (s, 1 H), 6.87 (d, J = 8.4 Hz, 1 H), 5.13-5.05 (m, 1 H), 2.73 (d, J = 7.0 Hz, 2 H); $^{13}C$ NMR ($DMSO-d_6$) δ 172.5, 168.0, 154.5, 143.6, 143.1, 129.0, 128.8, 127.4, 127.1, 126.8, 116.9, 50.4, 41.4. IR (KBr) 3343, 1693, 1661, 1649, 1604, 1543, 1414, 1239, 852, 762, 699. Anal. Calcd for $C_{17}H_{17}N_3O_4$-(0.84 $H_2O$): C, 59.62; H, 5.50; N, 12.27. Found: C, 59.62; H, 5.26; N, 12.18.

EXAMPLE 8

Preparation of N-(4-Sulfonamidophenyl)-N'-[3-(3-phenylpropionic acid)]urea

Methyl 3-isocyanato-3-phenylpropionate was prepared by the procedure of Example 7. To a solution of methyl 3-isocyanato-3-phenylpropionate (1.59 g, 7.75 mmol) in acetonitrile (50 mL) was added sulfanilamide (1.33 g, 7.75 mmol) in one portion with stirring. The resulting homogenous solution was allowed to stand for 3 weeks, during which time a white precipitate formed. Vacuum filtration yielded N-4-sulfonamidophenyl)-N'-[3-(methyl 3-phenylpropionate)]urea as a white solid (2.35 g, 80.5 %). mp 221-222 °C; $^{1}$H NMR (DMSO) δ 8.93 (s, 1 H), 7.63 (d, J = 8.7 Hz, 2 H), 7.49 (d, J = 8.6 Hz, 2 H), 7.38-7.18 (m, 5 H), 7.13 (s, 2 H), 6.93 (d, J = 8.4 Hz, 1 H), 5.11 (q, J = 7.5 Hz, 1 H), 3.52 (s, 3 H), 2.93-2.76 (m, 2 H). IR (KBr)Cm$^{-1}$ 3800-2800 (br), 1723, 1688, 1682, 1594, 1392, 1493, 1333, 1239, 1157, 1015, 837, 702, 607. Anal. Calcd for $C_{17}H_{19}N_3O_5S_1$: C, 54.10; H, 5.07; N, 11.13; S, 8.50. Found: C, 54.36; H, 5.22; N, 10.91; S, 8.56.

To a stirred solution of methyl ester from above (2.00 g, 5.30 mmol) in methanol/water (3:2, 50 mL) was added LiOH (0.22 g, 5.30 mmol) in water (5 mL) over 4 hr. The resulting suspension was filtered. The filtrate was washed with methylene chloride (3 X 15 mL), and then acidified (1 N HCl) to pH 2 yielding N-(4-sulfonamidophenyl)-N'-[3-(3-phenylpropionic acid)]urea as a white solid (1.08 g, 56 %): mp 165-167 °C with decomposition; $^{1}$H NMR (DMSO-d$_6$) δ 8.96 (s, 1 H), 7.62 (d, J = 8.7 Hz, 2 H), 7.47 (d, J = 8.8 Hz, 2 H), 7.39-7.18 (m, 5 H), 7.13 (s, 2 H), 6.93 (d, J = 8.3, 1 H), 5.08 (q, J = 7.8 Hz, 1 H), 2.73 (d, J = 7.3 Hz, 2 H); $^{13}$C NMR (DMSO-d$_6$) δ 40.87, 49.97, 116.83, 126.31, 126.75, 127.00, 128.32, 136.14, 142.54, 143.41, 154.00, 172.04; IR (KBr) cm$^{-1}$ 3650-2800 (br), 1883, 1840, 1592, 1541, 1326, 1155. Anal. Calcd for $C_{16}H_{17}N_3O_5S_1$(1 $H_2O$): C, 50.39; H, 5.02; N, 11.02; S, 8.41. Found: C, 50.75; H, 4.96; N, 10.90; S, 8.31.

EXAMPLE 9

Preparation of N-(4-Carbomethoxyphenyl)-N'-[3-(3-phenylpropionic acid)]urea

A solution of 3-amino-3-phenylpropionic acid (3.19 g, 19.3 mmol) and NaOH (0.77 g, 19.3 mmol) in water/acetonitrile (1:1, 20 mL) was added in three portions over 15 min to a vigorously stirred solution of 4-methoxycarbonylphenyl isocyanate (3.00 g, 19.3 mmol) in acetonitrile (20 mL). The acetonitrile was removed by rotary evaporation and the resulting aqueous solution was washed with ethyl acetate (2 X 25 mL). After acidification of the aqueous phase (pH 2) with 1 N HCl, the desired urea precipitated (3.61 g, 55 %) as a white solid: mp 111-112 °C with decomposition; $^{1}$H NMR (DMSO-d$_6$) δ 9.01 (s, 1 H), 7.79 (d, J = 8.7 Hz, 2 H), 7.47 (d, J = 8.7 Hz, 2 H), 7.40-7.17 (m, 5 H), 6.95 (d, J = 8.4 Hz, 1 H), 5.10 (q, J = 7.2 Hz, 1 H), 3.76 (s, 3 H), 2.75 (m, 2 H); $^{13}$C NMR (DMSO-d$_6$) δ 172.48, 166.41, 154.39, 145.40, 142.99, 130.79, 128.74, 127.41, 126.76, 122.28, 117.16, 52.08. 50.41, 41.31; IR (KBr) cm$^{-1}$ 3600-2400 (br), 1712, 1657, 1594, 1548, 1436, 1411, 1285, 1245, 1176, 1113, 765, 700. Anal. Calcd for $C_{18}H_{18}N_2O_5$: C, 63.15; H, 5.30; N, 8.18. Found: C, 63.09; H, 5.45; N, 7.89.

EXAMPLE 10

Preparation of N-4-(Carboethoxyphenyl)-N'-[3-(3-(3-pyridyl)propionic acid)]urea

To a solution of $NaHCO_3$ (2.13 g, 25.3 mmol) in water (5 mL) was added 3-amino-3-(3-pyridyl)propionic acid (4.21 g, 25.3 mmol). The resulting solution was concentrated (5 mm vacuum) to dryness and ethanol was added (20 mL). This suspension was concentrated (5 mm vacuum) and the ethanol treatment and concentration was repeated a second time. The white solid thus formed was suspended in methanol (50 mL) and carboethoxyphenyl isocyanate (4.84 g, 25.3 mmol) added in one portion which resulted in the formation of a clear solution. After 4 hr, the solution was concentrated to 15 mL and additional carboethoxyphenyl isocyanate (1.2 g, 6.3 mmol) was added. Concentration of this solution (5 mm vacuum) afforded a white solid. Water (10 mL) was added to the solid and after vigorous stirring, the suspension was filtered. The filtrate was washed with methylene chloride (2 X 5 mL) and concentrated (5 mm vacuum) providing a white foam. This material was purified by reverse phase high pressure liquid chromatography (100 % water) and afforded the desired product as sodium salt (white solid): mp 190-195 °C with decomposition; $^{1}$H NMR (DMSO-d$_6$) δ 10.92 (s, 1 H), 8.91 (d, J = 6.0 Hz, 1 H), 8.65 (s, 1 H), 8.40 (d, J = 4.4 Hz, 1 H), 7.79 ( d, J = 8.8 Hz, 3 H), 7.63 (d, J = 8.8 Hz, 2 H), 7.31 (d of d [J = 4.8 and 7.7 Hz, 1 H), 5.19 (q J = 6.4 Hz, 1 H), 4.26 (q, J = 7.1 Hz, 2 H), 2.60 (m, 2 H), 1.30 (t, J = 7.2 Hz, 3 H); $^{13}$C NMR (DMSO-d$_6$) δ 175.66, 165.98, 155.25, 148.51, 147.57, 146.43, 141.05, 134.22, 130.36, 123.51, 121.47, 116.98, 60.35, 50.02, 45.10, 14.55;

IR (KBr) $cm^{-1}$ 3700-2600 (br), 1693, 1597, 1547, 1411, 1285, 1176. Anal. Calcd for $C_{18}H_{18}N_3O_5Na_1$(1.3 $H_2O$): C, 53.68; H, 5.16; N, 10.43. Found: C, 53.66; H, 4.85; N, 10.44.

EXAMPLE 11

Preparation of N-(4-Carbamoylphenyl)-N'-[3-(3-(3-pyridyl)propionic acid)]urea

Procedure A:

A solution of 3-amino-3-(3-pyridyl)propionic acid (4.21 g, 25.3 mmol) in water (10 mL) was treated with NaOH (1.01 g, 25.3 mmol) forming the sodium salt. This solution was added to a solution of 4-carboethoxyphenyl isocyanate (7.62 g, 39.9 mmol) in acetonitrile (60 mL). After stirring for 2 days, less than 5 % of the starting amino acid remained unreacted as determined by HPLC. The resulting suspension was filtered. The remaining acetonitrile was removed by vacuum evaporation and water (20 mL) added to the solution. The resulting aqueous solution was washed with ethyl acetate (3 X 10 mL) and concentrated (5 mm) yielding the crude product as a gummy oil. $^1H$ NMR (DMSO-$d_6$) δ 10.78 (s, ca. 1 H), 8.73 (d, J = 5.7 Hz, ca. 1 H), 8.58-8.52 (m, 1 H), 8.43-8.32 (m, 1 H), 7.75-7.65 (d, J = 8.6 Hz, 3 H). 7.53 (d, J = 8.6 Hz, 2 H), 7.35-7.20 (m, 1 H), 5.00 (q, J = 6.7 Hz, 1 H), 4.20 (q, J = 7.6 Hz, 2 H), 1.52-2.40 (m, 2 H), 1.24 (t, J = 7.6 Hz, 3 H).

To the above crude product (2.5 g, 7.0 mmol) in a Parr Type high pressure reactor was added $NH_4OH$ (150 mL, 14.8 M) and the solution heated to 80 °C for 4.5 hr. The resulting solution was concentrated yielding a syrup. The syrup was chromatographed on an HPLC system using Whatman Partisil 20, $C_{18}$ packing using 100 % $H_2O$. When the desired product began to elute, the solvent strength was increased to acetonitrile:water (2.5:97.5). The fractions containing the product were combined and concentrated (5 mm) until only about 25 mL of solution remained. This solution was lyophilized yielding the desired product as a white solid (0.610 g, 25 %), obtained as a mixture of sodium and ammonium salts: $^1H$ NMR (DMSO-$d_6$) δ 9.52 (s, 1 H), 8.53 (s, 1 H), 8.36 (d, J = 5.7 Hz, 1 H), 8.17 (d, J = 5.7 Hz, 1 H), 7.78-7.62 (m, 4 H), 7.43 (d, J = 8.6 Hz, 2 H), 7.29-7.21 (m, 1 H), 7.05 (s, 1 H), 4.97 (q, J = 6.7 Hz, 1 H), 2.43 (m, 2 H); IR (KBr) $cm^{-1}$ 3600-2800 (br), 1663, 1585, 1539, 1412, 1396, 1328, 1316, 1242, 1185, 1115, 851, 769, 711.

Procedure B:

Conversion of N-(4-Cyanophenyl)-N'-[3-(3-(3-pyridyl)propionic acid)] urea to sodium salt of N-4-Carbamoylphenyl-N'-[3-(3-(3-pyridyl)propionic acid)] urea:

Hydrogen peroxide (30%, 3.45 mL, 9.60 mmol) was added to a stirred suspension of N-(4-cyanophenyl)-N'-[3-(3-(3-pyridyl)propionicacid)]urea was prepared as detailed in Example 5 and 2.90 g, 9.60 mmol was placed in ethanol (6.9 mL), water (6.9 mL) and sodium hydroxide (6N, 2.07 mL, 12.42 mmol). The reaction mixture was stirred for 15 min at room temperature until the contents of the flask became clear and the evolution of gas (oxygen) stopped. Sodium bisulfite (2g) was added to the reaction mixture to destroy excess hydrogen peroxide. The reaction mixture was concentrated in vacuo at room temperature and then chromatographed (reverse phase HPLC, water as the eluant). Pure fractions were combined and lyophilized to afford 1.90 g (62%) of the desired product as a white crystalline powder. $^1H$ NMR ($D_2O$) δ 2.70 (d, 2H, J = 7.3 Hz), 5.10 (t, 1H, J = 7.1 Hz), 7.33 and 7.68 (AB quartet 4H, J = 7.6 Hz), 7.38-7.43 (m, 1H), 7.84 (d, 1H, J = 8.0 Hz), 8.39 (d, 1H, J = 4.4 Hz), 8.51 (s, 1H). Anal Calcd for $C_{16}H_{15}N_4NaO_4$(1.5$H_2O$): C, 50.93; H, 4.8: N, 14.84. Found: C, 50.83; H, 4.20; N, 14.27

EXAMPLE 12

Preparation of N-(4-Carboxyphenyl)-N'-[3-(3-(3-pyridyl)propionic acid)]urea

To a stirred solution of the ethyl ester produced in Example 10 (3.00 g, 7.91 mmol) in water was added NaOH (8.7 mL, 8.7 mmol, 1N). After 20 hr, no starting materials remained as determined by HPLC. The reaction mixture was concentrated (5 mm vacuum), dissolved in water (5 mL), filtered (Acrodisc-HPLC filter), and purified by high pressure liquid chromatography (Whatman partisil-20, ODS-3). Concentration (5 mm vacuum) to 50 mL followed by lyophilization afforded the desired diacid as a white solid, as the disodium salt; $^1H$ NMR ($D_2O$ with 5 % DMSO-$d_6$) δ 8.40 (s, 1 H), 8.19 (s, 1 H), 7.80-7.55 (m, 3 H), 7.28-7.07 (m, 3 H), 5.15-4.95 (m, 1 H), 2.73-2.48 (m, 2 H); $^{13}C$ NMR ($D_2O$ with 5 % DMSO-$d_6$) δ 178.50, 175.05,

156.35, 147.45, 146.82, 141.31, 138.78, 135.18, 130.43, 130.17, 124.33, 118.66, 50.08, 43.97; IR (KBr) $cm^{-1}$ 3700-2400 (br), 1688, 1603, 1387, 1311, 1239, 792, 702. Anal. Calcd for $C_{16}H_{13}N_3O_5Na_2$ (3.51 $H_2O$): C, 44.01; H, 4.63; N, 9.62. Found: C, 44.02; H, 4.15; N, 9.71.

EXAMPLE 13

Preparation of N-(4-Iodophenyl)-N'-[3-(3-phenylpropionic acid)]urea

To a solution of 4-iodophenyl isocyanate (2.45 g, 10.0 mmol) in 30 mL of acetonitrile was added a solution of 3-amino-3-phenylpropionic acid (1.67 g, 10.1 mmol) and sodium hydroxide (0.404 g, 10.1 mmol) in 10 mL of 1:1 acetonitrile-water. Precipitation of a white solid made the reaction suspension difficult to stir, and it was diluted with 10 mL of acetonitrile and 10 mL of water. The milky white solution was stirred at room temperature for 16.5 h, and then the acetonitrile removed at reduced pressure. The aqueous residue was diluted to 150 mL with water, and then extracted with three portions of ethyl acetate. The aqueous solution was made basic with 1 N sodium hydroxide, then filtered to remove a white solid. The solid was washed with water and then dried in vacuo at 60 °C. This material, 1.74 g (40%) was identified as the sodium salt of the desired product. The filtrate was acidified to pH 1 with conc. hydrochloric acid. The precipitate was filtered, washed with water and ether, then dried in vacuo at 60 °C to give 1.15 g (28%) of a white solid: mp: 208-209 °C; $^1H$ NMR (300 MHz; DMSO-$d_6$) $\delta$ 8.70 (s, 1 H), 7.52-7.20 (AB, 4 H, $J_{AB}$=8.8 Hz), 7.33-7.28 (m, 5 H), 6.83 (d, 1 H, J=8.4 Hz), 5.12-5.08 (m, 1 H), and 2.76-2.73 (m, 2 H); $^{13}C$ NMR (75.5 MHz; DMSO-$d_6$) $\delta$ 172.2, 154.3, 142.8, 140.3, 137.3, 128.4, 127.1, 126.4, 120.1, 83.9, 50.0, and 41.1; IR (KBr): 3338, 3304, 3064, 3032, 2928, 1705, 1651, 1592, 1547, 1486, 1398, 1314, 1240, and 712 $cm^{-1}$. Analysis: Calculated for $C_{16}H_{15}IN_2O_3(H_2O)_{0.37}$: C 46.08; H 3.81; N 6.72. Found: C 46.07; H 3.73; N 6.75.

EXAMPLE 14

Preparation of N-(4-Chlorophenyl)-N'-[3-(3-phenylpropionic acid)]urea

To a solution of 4-chlorophenyl isocyanate (1.54 g, 10.0 mmol) in 35 mL of acetonitrile was added a solution of 3-amino-3-phenylpropionic acid (1.67 g, 10.1 mmol) and sodium hydroxide (0.406 g, 10.2 mmol) in 10 mL of 1:1 acetonitrile-water. The homogeneous solution was stirred at room temperature for 1.5 h, and then the acetonitrile removed at reduced pressure. The aqueous solution was diluted to 150 mL with water, extracted with two portions of ethyl acetate, and then acidified to pH 1 with conc. hydrochloric acid. The precipitate was filtered, washed with water, and then dried in vacuo to give 2.82 g (88%) of a white solid: mp 185-186 °C; $^1H$ NMR (300 MHz; DMSO-$d_6$) $\delta$ 8.74 (s, 1 H), 7.41-7.22 (AB, 4 H, $J_{AB}$=8.8 Hz), 7.37-7.29 (m, 5 H), 6.84 (d, 1 H, J=8.4 Hz), 5.16-5.08 (m, 1 H), and 2.77-274 (m, 1 H); $^{13}C$ NMR (75.5 MHz; DMSO-$d_6$) $\delta$ 172.2, 154.4, 142.8, 139.4, 128.6, 128.5, 127.1, 126.4, 124.8, 119.2, 50.1 and 41.1; IR (KBr): 3336, 3304, 3064, 3032, 2928, 1706, 1652, 1595, 1553, 1493, 1398, 1312, 1240, and 704 $cm^{-1}$. Analysis: Calculated for $C_{16}H_{15}ClN_2O_3(H_2O)$: C, 60.05; H, 4.77; N, 8.75. Found: C, 60.05; H, 4.74; N, 8.83.

EXAMPLE 15

Preparation of N-(3-Chlorophenyl)-N'-[3-(3-phenylpropionic acid)]urea

To a solution of 3-chlorophenyl isocyanate (1.54 g, 10.0 mmol) in 35 mL of acetonitrile was added a solution of 3-amino-3-phenylpropionic acid ( 1.67 g, 10.1 mmol) and sodium hydroxide (0.436 g, 10.9 mmol) in 10 mL of 1:1 acetonitrile-water. The homogeneous solution was stirred at room temperature for 3 h, then concentrated at reduced pressure to afford a yellow oil. This material was dissolved in 100 mL of water, extracted with two portions of methylene chloride, and then acidified to pH 0-1 with conc. hydrochloric acid. The precipitate was filtered, washed with water, and dried in vacuo at 60 °C to give 2.86 g (90%) of a white solid: mp 172-173 °C; $^1H$ NMR (300 MHz, DMSO-$d_6$) $\delta$ 8.84 (s, 1 H), 7.67 (s, 1 H), 7.37-7.29 (m, 4 H), 7.24-7.15 (m, 3 H), 6.91 (d, 2 H, J=7.8 Hz), 5.18-5.11 (m, 1 H), and 2.79-2.76 (m, 2 H); $^{13}C$ NMR (75.5 MHz; DMSO-$d_6$) $\delta$ 172.3, 154.4, 142.8, 142.0, 133.4, 130.4, 128.5, 127.2, 126.5, 121.0, 117.1, 116.2, 50.2, and 41.1; IR (KBr): 3392, 3064, 3032, 2928, 1717, 1653, 1592, 1552, 1483, 1424, and 700 $cm^{-1}$. Analysis: Calculated for $C_{16}H_{15}ClN_2O_3$: C, 60.29; H, 4.74; N, 8.79. Found: C, 60.34; H, 4.70; N, 8.82.

EXAMPLE 16

Preparation of N-(4-Methylphenyl)-N'-[3-(3-phenylpropionic acid)]urea

To a solution of 4-methylphenyl isocyanate (1.33 g, 10.0 mmol) in 35 mL of acetonitrile was added a solution of 3-amino-3-phenylpropionic acid (1.67 g, 10.1 mmol) and sodium hydroxide (0.434 g, 10.9 mmol) in 10 mL of 1:1 acetonitrile-water. The homogeneous solution was stirred at room temperature for 2.5 h, then partially concentrated at reduced pressure. The aqueous solution was diluted with 200 mL of water, extracted with two portions of ethyl acetate, and then acidified to pH 0-1 with conc. hydrochloric acid. The precipitate was filtered, washed with water, and dried in vacuo at 60 °C to give 2.86 g (96%) of a white solid: mp 169-170 °C; $^1H$ NMR (300 MHz; DMSO-$d_6$) δ 8.49 (s, 1 H), 7.38-7.20 (m, 5 H), 7.29-7.00 (AB, 4 H, $J_{AB}$ = 8.3 Hz), 6.75 (d, 1 H, J = 8.5 Hz), 5.19-5.12 (m, 1 H), 2.78-2.75 (m, 2 H), and 2.19 (s, 3 H); $^{13}C$ NMR-(75.5 MHz; DMSO-$d_6$) δ 172.3, 154.7, 143.1, 137.9, 130.2, 129.3, 128.5, 127.1, 126.5, 117.9,50.1, 41.3, and 20.5; IR (KBr): 3392, 3032, 2928, 1718, 1646, 1601, 1555, 1514, 1408, 1312,1240, 1195, 816, and 712 $cm^{-1}$. Analysis: Calculated for $C_{17}H_{18}N_2O_3$: C, 68.44; H, 6.08; N, 9.39. Found: C, 68.38; H, 6.10; N, 9.37.

EXAMPLE 17

Preparation of N-(4-Trifluoromethylphenyl)-N'-[3-(3-phenylpropionic acid)]urea

To a solution of 4-trifluoromethylphenyl isocyanate (1.87 g, 10.0 mmol) in 35 mL of acetonitrile was added a solution of 3-amino-3-phenylpropionic acid (1.67 g, 10.1 mmol) and sodium hydroxide(0.414 g, 10.3 mmol)in 10 mL of 1:1 acetonitrile-water. The reaction mixture was stirred at room temperature for 4.5 h, then partially concentrated at reduced pressure. The aqueous solution was diluted with 150 mL of water and then acidified to pH 0-1 with conc. hydrochloric acid. The yellow solid that precipitated was filtered and washed with water. It was then dissolved in 150 mL of ether and extracted with three portions of aqueous sodium hydroxide. The aqueous solution was acidified to pH 0-1 with conc. hydrochloric acid. The precipitate was filtered, washed with water, and dried in vacuo at 60 °C to give 3.18 g (90%) of a white solid: mp 172-173 °C; $^1H$ NMR (300 MHz; DMSO-$d_6$) δ 9.12 (s, 1 H), 7.59-7.52 (AB, 4 H, $J_{AB}$ = 9 2 Hz), 7.38-7.20 (m, 5 H), 7.04 (d, 1 H, J = 8.5 Hz), 5.18-5.11 (m, 1 H), and 2.79-2.76 (m, 2 H); $^{13}C$ NMR (75.5 MHz; DMSO-$d_6$) δ 172.2, 154.3, 144.2, 142.8, 128.5, 127.2, 126.5, 126.1, 123.0, 121.4, 117.4, 50.2, and 41.1; IR (KBr): 3360, 3064, 3032, 2928, 1720, 1654, 1602, 1555, 1327, 1248, 1168, 1115, 1072, and 710 $cm^{-1}$. Analysis: Calculated for $C_{17}H_{15}F_3N_2O_3$ $(H_2O)_{0.36}$: C, 56.88; H, 4.42; N, 7.80; Found: C, 56.87; H, 4.27; N, 7.81.

EXAMPLE 18

Preparation of N-(4-Cyanophenyl)-N'-[3-(3-(4'-methoxyphenyl)propionic acid)]urea

To a solution of p-anisaldehyde (40.8 g, 300 mmol) in 100 mL of 95:5 ethanol-water was added ammonium acetate (46.2 g, 600 mmol). The reaction mixture was warmed to 45 °C, and then treated with malonic acid (31.2 g, 300 mmol) in one portion. The resulting suspension was heated at reflux for 18 h, allowed to cool to room temperature, and filtered. The precipitate was recrystallized from 3:1 ethanol-water to give 30.9 g (53%) of a white solid 3-amino-3-(4'-methoxyphenyl)propionic acid: mp 234-235 °C; $^1H$ NMR (300 MHz; HOAc-$d_4$) δ 7.45-6.95 (AB, 4 H, $J_{AB}$ = 8.6 Hz), 4.76 (dd, 1 H, J = 9.1, 5.2 Hz), 3.79 (s, 3 H), 3.24 (dd, 1 H, J = 17.3, 9.1 Hz), and 2.97 (dd, 1 H, J = 17.3, 5.2 Hz); $^{13}C$ NMR(75.5 MHz; HOAc-$d_4$) δ 176.2, 161.2, 129.7, 128.4, 115.1, 55.1, 52.8, and 38.9; IR (KBr): 3424, 2937, 2616, 1613, 1535, 1518, 1407, 1251, 1184, 1027, and 838 $cm^{-1}$. Analysis Calculated for $C_{10}H_{13}NO_3$: C, 61.53, H, 6.71; N, 7.18. Found: C, 61.86; H, 6.56; N, 7.10.

To a solution of 4-cyanophenyl isocyanate (1.44 g, 10.0 mmol) in 35 mL of acetonitrile was added a solution of 3-amino-3-(4'-methoxyphenyl)propionic acid (1.97 g, 10.1 mmol) and sodium hydroxide (0.430 g, 10.8 mmol) in 10 mL of 1:1 acetonitrile-water. The resulting milky white solution was stirred at room temperature for 4 h and then partially concentrated to remove the acetonitrile. The aqueous solution was diluted with 200 mL of water and acidified to pH 1.5 with conc. hydrochloric acid. The precipitate was filtered, washed with water and ether, and then dried in vacuo to give 2.60 g (77%) of an off white solid: mp 105-107 °C; $^1H$ NMR (300 MHz; DMSO-$d_6$) δ 9.09 (s, 1 H), 7.66-7.52 (AB, 4 H, $J_{AB}$ = 8.8 Hz), 7.28-6.87 (AB, 4 H, A = 7.24, B = 6.90, $J_{AB}$ = 8.7 Hz), 6.95 (d, 1 H, J = 8.4 Hz), 5.09-5.02 (m, 1 H), 3.71 (s, 3 H), and 2.81-2.67 (m, 2 H); $^{13}C$ NMR (75.5 MHz; DMSO-$d_6$)δ 172.2, 158.3,153.8, 144.8, 134.4, 133.2, 127.6, 119.5, 117.5,

113.7, 102.6, 55.1, 49.5, and 40.9; IR (KBr): 3360, 2225, 1716, 1675, 1593, 1537, 1514, 1319, 1250, 1233, 1176, 838, and 548 $cm^{-1}$. Analysis: Calculated for $C_{18}H_{17}N_3O_4$ $(H_2O)_{0.88}$: C, 60.87; H, 5.32; N, 11.83. Found: C, 60.84; H, 5.41; N, 12.04.

EXAMPLE 19

N-(4-Cyanophenyl)-N'-[3-(3-(3',4'-dimethoxyphenyl)propionic acid)]urea

To a solution of 3,4-dimethoxybenzaldehyde (16.6 g, 100 mmol) in 50 mL of 9:1 ethanol-water was added ammonium acetate (15.4 g, 200 mmol). The reaction mixture was warmed to 45 °C, and then treated with malonic acid (10.4 g, 100 mmol) in one portion. The suspension was heated at reflux for 16.5 h, then cooled and filtered. The precipitate was washed with several portions of ether and then dried in vacuo at 60 °C to yield 12.1 g (54%) of a white solid, 3-amino-3-(3',4'-dimethoxyphenyl)propionic acid: mp 216-217 °C; $^1H$ NMR (300 MHz; $D_2O$) δ 6.93 (s, 1 H), 6.90 (s, 2 H), 4.44 (dd, 1 H, J=8.0, 6.6 Hz), 3.72 (s, 3 H), 3.69 (s, 3 H), 2.75 (dd, 1 H, J=16.2, 6.6 Hz), and 2.64 (dd, 1 H, J=16.2, 8.0 Hz); $^{13}C$ NMR (75.5 MHz; AcOH-$d_4$) δ 176.1, 150.6, 150.2, 128.9, 120.9, 112.5, 111.6, 56.0, 53.2, and 39.0; IR (KBr): 3424, 2935, 2836, 1604, 1574, 1552, 1523, 1465, 1396, 1273, 1148, and 1025 $cm^{-1}$. Analysis: Calculated for $C_{11}H_{15}NO_4$: C, 58.66; H, 6.71; N, 6.22. Found: C, 58.42; H, 6.63; N, 6.15.

To a solution of 4-cyanophenyl isocyanate (1.08 g, 7.50 mmol) in 40 mL of acetonitrile was added a solution of 3-amino-3-(3',4'-dimethoxyphenyl)propionic acid (1.71 g, 7.58 mmol) and sodium hydroxide (0.309 g, 7.72 mmol) in 5 mL of water. The reaction mixture was stirred for 3.5 h at room temperature and then partially concentrated at reduced pressure. The aqueous solution was diluted with 100 mL of water and then acidified to pH 2 with conc. hydrochloric acid, resulting in formation of a gum. The liquid was decanted and the gummy residue was dissolved with aqueous sodium hydroxide. The basic solution was washed with portions of ether and methylene chloride, then acidified to pH 2 with conc. hydrochloric acid, resulting in formation of a gum. The aqueous solution was diluted with 15 mL of methanol and then warmed gently until the gum solidified. The precipitate was filtered, washed with water, and dried in vacuo at 60 °C to give 2.00 g (72%) of a white solid: mp 148-150 °C;$^1H$ NMR (300 MHz; DMSO-$d_6$) δ 12.30 (br s, 1 H), 9.11 (s, 1 H), 7.66-7.53 (AB, 4 H, $J_{AB}$=8.8 Hz), 6.99-6.83 (m, 4 H), 5.09-5.02 (m, 1 H), 3.74 (s, 3 H), 3.71 (s, 3 H), and 2.76-2.73 (m, 2 H); $^{13}C$ NMR (75.5 MHz; DMSO-$d_6$) δ 172.2, 153.8, 148.6, 147.9, 144.9, 135.0, 133.2, 119.5, 118.3, 117.5, 111.7, 110.5, 102.6, 55.6, 49.9, and 41.1; IR (KBr): 3360, 2224, 1704, 1594, 1518, 1411, 1319, 1233, 1145, 1024, 848, and 552 $cm^{-1}$. Analysis: Calculated for $C_{19}H_{19}N_3O_5$ $(H_2O)_{0.86}$: C, 59.30; H, 5.43; N, 10.92. Found: C, 59.27; H, 5.07; N, 10.88.

EXAMPLE 20

Preparation of N-(4-Cyanophenyl)-N'-[3-(3-phenylpropionic acid)]thiourea.

To a stirred suspension of 4-cyanophenyl isothiocyanate (1.60 g, 10.0 mmol) and 3-amino-3-phenylpropionic acid (1.65 g, 10.0 mmol) in 50 mL of acetonitrile was added 10 mL of 1 N NaOH. The clear yellow solution which immediately formed was stirred overnight and the solvent then removed under reduced pressure. The residue was dissolved in 50 mL of 1:1 ethyl acetate/water and the aqueous layer was extracted twice with 50 mL ethyl acetate. The product was precipitated from the aqueous layer as a gum after adjusting the pH to 2.5 with 4 N HCl. The gummy product was stirred overnight in water to produce a fluffy white solid. The solid was isolated by filtration and dried to yield 2.65 g (82%) of the desired product as a off-white powder: IR (KBr) $cm^{-1}$ 3320, 3150, 2235, 1733, 1604, 1542, 1519, 1509,1169; $^1H$ NMR (DMSO-$d_6$) δ 10.2 (s, 1H), 8.7 (d, 1H, J=8.3Hz), 7.7 (dd, 4H, J=8.3, J=24Hz), 7.2-7.5 (m, 5H), 5.8 (q, 1H, J=7.3Hz), 2.9 (dd, 2H, J=7.3, J=16Hz); $^{13}C$ NMR (DMSO-$d_6$) δ 184.6, 177.0, 149.3, 146.3, 137.8, 133.4, 132.3, 131.9, 126.3, 124.2, 109.9, 59.1. Anal. Calc. for $C_{17}H_{15}N_3SO_2$: C,62.75; H,4.65; N,12.91. Found: C,62.60; H,4.78; N,12.61.

EXAMPLE 21

Preparation of N-(4-Methoxycarbonylphenyl)-N'-[3-(3-phenylpropionic acid)]thiourea

To a stirred suspension of 4-methoxycarbonylphenyl isothiocyanate (1.93 g, 10.0 mmol) and 3-amino-3-phenylpropionic acid (1.65 g, 10.0 mmol) in 60 mL of acetonitrile was added 10 mL of 1 N NaOH. The yellow solution was stirred for one hour before the solvent was removed under vacuum. The residue was

dissolved in 200 mL of 50/50 ethyl acetate:water and the aqueous phase extracted with ethyl acetate (2 x 100 mL). The product was separated from the aqueous layer as a gum after adjusting the pH to 2 with 1 N HCl. The gum was stirred in water over the weekend and the product (2.0 g, 55%) isolated by filtration as a fine white powder: mp 144-6 °C; $^1H$ NMR (DMSO-$d_6$) δ 10.0 (s, 1H), 8.6 (s, 1H), 7.9 (d, 2H, J = 8.7Hz) 7.4 (m, 5H), 5.9 (q, 1H, J = 6.8Hz), 3.8 (s, 1H), 2.9 (dd, 2H, J = 6.8, J = 16.5Hz); $^{13}C$ NMR (DMSO-$d_6$) δ 184.2, 176.6, 170.5, 148.9, 145.9, 134.5, 132.9, 131.7, 131.4, 128.6, 125.4, 58.6, 56.6, 44.6. Anal. Calcd for $C_{18}H_{18}N_2O_4S(0.25\ H_2O)$: C,59.45; H,5.15; N,7.70. Found: C,59.44; H,5.06; N,7.62.

EXAMPLE 22

Preparation of N-(4-Cyanophenyl)-N'-[3-(3-(4-pyridylpropionic acid)]urea Sodium salt

To a stirred suspension of 3-amino-3-(4-pyridyl)propionic acid (0.17 g, 1.0 mmol) and 4-cyanophenyl isocyanate (0.45 g, 3.0 mmol) in 25 mL of acetonitrile was added 1.0 mL of 1 N NaOH and 5 mL of water. The clear solution was stirred for one hour before the solvents were removed at reduced presssure. The residue was dissolved in 75 ml of 50/50 ethyl acetate:water and the aqueous phased washed with ethyl acetate (2 x 50mL). The crude product (0.32 g) was isolated by lyophilization of the aqueous phase and purified by reverse phase chromatography to yield 0.12 g (36%) of a white powder: $^1H$ NMR (DMSO-$d_6$) δ 9.25 (bs, 1H), 8.4 (d, 2H, 5.8Hz), 7.7 (d, 2H, J = 8.7Hz), 7.5 (d, 2H, J = 8.7Hz), 7.3 (d, 2H, J = 5.8Hz), 5.0 (q, 1H, J = 5.8Hz), 2,4 (m, 2H); $^{13}C$ NMR δ 174.2, 155.0, 154.6, 149.2, 146.5, 132.7, 121.5, 119.6, 117.3, 101.2, 51.5, 44.6.

EXAMPLE 23

Preparation of N-(4-Carboxyphenyl)-N'-[3-(3-phenylpropionic acid)]urea

To a stirred solution of NaOH (0.224 g, 5.60 mmol) in 20 mL of 1/1 MeOH/water was added to the urea prepared in Example 1. (0.500 g, 1.40 mmol). After 3 h, the reaction mixture was partially concentrated to remove the MeOH. The reaction mixture was diluted to a volume of 50 mL with water and acidified with 6 mL of 1 N HCl. The precipitate was isolated by filtration and air-dried to afford 0.44 g (96%) of the urea as a white powder: mp 190-195 °C; $^1H$ NMR (DMSO-$d_6$) δ 12.43 (br s, 2 H), 9.0 (s, 1 H), 7.9-7.74 (m, 2 H), 7.55-7.2 (m, 7 H), 6.96 (d, J = 8.4 Hz, 2 H), 5.2-5.05 (m, 1 H), 2.9-2.7 (m, 2 H); $^{13}C$ NMR (DMSO-$d_6$) δ 172.0, 167.0, 153.9, 144.6, 142.6, 130.5, 128.3, 127.0, 126.3, 122.9, 116.6, 49.9, 40.8; IR(KBr) $cm^{-1}$ 3460, 3080, 3040, 1700, 1590, 1500, 1390, 1310, 1280, 1240, 1175. Anal. Calcd for $C_{17}H_{16}N_2O_5$-(0.13 $H_2O$): C, 61.72; H, 4.96; N, 8.47. Found: C, 61.71; H, 4.87; N, 8.73.

EXAMPLE 24

Preparation of N-(Phenyl)-N'-[3-(3-phenylpropionic acid)]urea

The urea was prepared analogously to N-(4-bromophenyl)-N'-(2-carboxy-1-phenylethyl)urea except phenyl isocyanate was substituted for 4-bromophenyl isocyanate to afford 2.69 g (91%) of the urea as a powder: mp 179-180 °C; $^1H$ NMR (DMSO-$d_6$) δ 12.30 (br s, 1 H, NH), 8.58 (s, 1 H), 7.6-7.1 (m, 8 H), 7.0-6.75 (m, 2 H), 5.17-5.10 (overlapping dt, 1 H), 2.9-2.7 (m, 2 H); $^{13}C$ NMR (DMSO-$d_6$) δ 172.1, 154.4, 142.9, 140.3, 128.7, 128.4, 126.9, 126.3, 121.2, 117.6, 49.9, 41.1; IR (KBr) $cm^{-1}$ 3360, 3060, 3020, 1718, 1640, 1600, 1560, 1500, 1460, 1400, 1310, 1240. Anal. Calcd for $C_{16}H_{16}N_2O_3$: C, 67.59; H, 5.67; N, 9.85. Found: C, 67.56; H, 5.58; N, 9.76.

EXAMPLE 25

Preparation of N-(4-Formylphenyl)-N'-[3-(3-phenylpropionic acid)]urea

To a stirred solution (slightly cloudy) of 1,1'-carbonyldiimidazole (5.27 g, 32.5 mmol) and imidazole (3.32 g, 48.7 mmol) in 50 mL of dry THF cooled in an ice bath was added a solution of methyl 3-amino-3-phenylpropionate (5.82 g, 32.5 mmol) in 10 mL of THF over 15 minutes. The reaction solution was stirred an additional 15 minutes, then a solution of 4-aminobenzyl alcohol (4.00 g, 32.5 mmol) in 25 mL of THF was rapidly added. After an additional 30 minutes, the cooling bath was removed and the reaction mixture was stirred for 17 hours. The reaction mixture was then concentrated, the residue dissolved in 100 mL of $CH_2Cl_2$

and washed with water (100 mL). The aqueous wash was extracted with $CH_2Cl_2$ (50 mL) and the organic layers combined, dried ($MgSO_4$), and concentrated to afford 9.27 g of crude product. The crude product was purified by flash chromatography (silica gel, 4-6% MeOH/$CH_2Cl_2$) to afford 3.5 g (33%) of N-(4-hydroxymethylphenyl)-N'-[3-(methyl 3-phenyl-propionate)]urea as a very pale yellow solid: mp 108-118 °C; TLC (1/9 $CH_3OH/CH_2Cl_2$, UV) $R_f$ = 0.44; $^1$H NMR (DMSO-$d_6$) $\delta$ 7. 69 (s, 1 H, NH), 7.03, 7.98 (AB quartet, J = 8.6 Hz, 4 H), 7.3-7.1 (m, 5 H), 6.48 (d, J = 8.3 Hz, 1 H, NH), 5.35-5.2 (m, 1 H), 4.38 (s, 2 H, $CH_2O$), 3.5 (s, 3 H, $CO_2CH_3$), 2.85-26 (m, 2 H, $CH_2$); $^{13}$C NMR (DMSO-$d_6$) $\delta$ 171.7, 155.5, 141.3, 138.1, 135.2, 128.6, 127.7, 127.4, 126.1, 119.8, 64.4, 51.8, 50.6, 41.0; IR (KBr) $cm^{-1}$ 3340 (br), 1735, 1690, 1660, 1600, 1550, 1513, 1440, 1418. Anal. Calcd for $C_{18}H_{20}N_2O_4$: C, 65.84; H, 6.14; N, 8.53. Found: C, 65.94; H, 6.20; N, 8.84.

To a stirred solution of N-(4-hydroxymethylphenyl)-N'-[3-(methyl 3-phenylpropionate)]urea (2.30 g, 7.01 mmol) in 230 mL of $CH_2Cl_2$ was added $MnO_2$ (3.00 g, 34.5 mmol) as a solid in one portion. The reaction suspension was stirred for 44 h, then filtered through celite. The filtrate was concentrated and the residue purified by flash chromatography (3/7 EtOAc/hexane, silica gel) to afford 1.14 g (50%) of the desired N-(4-Formylphenyl)-N'-[3-(methyl 3-phenylpropionate)]urea. An additional 0.518 g (23%) of material was obtained from copious washing of the celite cake with $CH_2Cl_2$, $CH_3CN$, and EtOH followed by flash chromatography purification: TLC (0.5/9.5 $CH_3OH/CH_2Cl_2$, UV) $R_f$ = 0.38; $^1$H NMR (DMSO-$d_6$) $\delta$ 9.79 (s, 1 H, CHO), 9.11 (s, 1 H), 7.76 (d, 2 H, J = 8.6 Hz), 7.57 (d, 2 H, J = 8.6 Hz), 7.4-7.2 (m, 5 H), 7.02 (d, 1 H, J = 8.4 Hz, CHNH), 5.18-5.10 (m, 1 H, CH), 3.54 (s, 3 H, $CO_2CH_3$), 2.95-2.82 (m, 2 H); $^{13}$C NMR (DMSO-$d_6$) $\delta$ 191.3, 170.9, 153.8, 146.2, 142.1, 131.1, 129.6, 128.4, 127.2, 126.3, 117.0, 51.5, 50.0, 40.6; IR (KBr) $cm^{-1}$ 3370, 3320, 1727, 1687, 1669, 1595, 1560, 1544, 1435, 1365, 122, 1165; TLC (3/7 EtOAc/hexane) $R_f$ = 0.48. Anal. Calcd for $C_{18}H_{18}N_2O_4$: C, 65.70; H, 5.61; N, 8.51. Found: C, 65.68; H, 5.47; N, 8.12.

To a stirred suspension of N-(4-formylphenyl)-N-[3-(methyl 3-phenylpropionate)]urea (1.14 g, 3.49 mmol) in 230 mL of MeOH and 50 mL of water was added 14 mL of 1 N NaOH (14 mmol). The reaction mixture became homogeneous after 1 h. After 3.5 hours, the reaction solution was concentrated to remove the MeOH, and diluted to a total volume of 250 mL with water. This solution was washed with EtOAc (100 mL). The aqueous layer was partially concentrated to remove traces of EtOAc and the pH adjusted to 1 with 17 mL of 1 N HCl. A gum formed and the suspension was stirred overnight. The gum had solidified and the resulting solid was isolated by filtration. The white powder was dried in vacuo (<0.2 mm, 40 °C) to afford 1.06 g (97%) of the desired urea : mp 145-148 °C; $^1$H NMR (DMSO-$d_6$) $\delta$ 12.35 (br s, 1 H), 9.79 (s, 1 H, CHO), 9.15 (s, 1 H, NH), 7.76 (d, 2 H, J = 8.5 Hz), 7.57 (d, 2 H, J = 8.5 Hz), 7.45-7.2 (m, 5 H, Ph), 7.04 (d, 1 H, J = 8.4 Hz), 5.2-5.05 (m, 1 H), 2.9-2.7 (m, 2 H); $^{13}$C NMR (DMSO-$d_6$) $\delta$ 191.5, 191.0, 172.0, 153.8, 146.2, 142.5, 131.1, 129.6, 128.4, 126.4, 117.0, 50.0, 40.8; IR (KBr) $cm^{-1}$ 3400, 3360, 3060, 1720, 1690, 1673, 1660, 1560, 1540, 1166. Anal. Calcd for $C_{17}H_{16}N_2O_4$-(0.11 $H_2O$): C, 64.93; H, 5.21; N, 8.91. Found: C, 64.90; H, 5.10; N, 8.85.

EXAMPLE 26

Preparation of N-(4-Hydroxymethylphenyl-N'-[3-(3-phenylpropionic acid)]urea

To a stirred solution of N-(4-hydroxymethylphenyl)-N'-[3-(methyl 3-phenylpropionate)]urea prepared as in Example 25, (0.500 g, 1.52 mmol) in 25 mL of $CH_3OH$ was added 5 mL of 1 N NaOH and 5 mL of water. Reaction progress was monitored by HPLC. After 1.5 h, the reaction mixture was partially concentrated to remove the $CH_3OH$. The reaction mixture was then diluted with 20 mL of water and acidified with 5 mL of 1 N HCl. A gum formed upon acidification. The reaction mixture was diluted with 5 mL of $CH_3OH$ and the reaction mixture was stirred overnight. The resulting slurry was filtered to yield after air-drying 0.35 g (73%) of the urea as a flocculent white powder: mp 138-140 °C; $^1$H NMR (DMSO-$d_6$) $\delta$ 12.3 (br s, 1 H, COOH), 8.54 (s, 1 H, NH), 7.45-7.1 (m, 9 H, Ar and Ph), 6.75 (d, J = 8.5 Hz, 1 H), 5.11( apparent q, 1 H), 5.01 (br s, 1 H), 4.38 (s, 2 H), 2.85-2.65 (m, 2 H); $^{13}$C NMR (DMSO-$d_6$) $\delta$ 172.1, 154.4, 142.9, 139.0, 135.2, 128.4, 127.2, 127.0, 126.4, 117.4, 62.8, 50.0, 41.1; IR (KBr) $cm^{-1}$ 3400, 3340, 1710, 1660, 1550, 1420, 1320, 1240. Anal. Calcd for $C_{17}H_{18}N_2O_4$: C, 64.96; H, 5.77; N, 8.91. Found: C, 64.70; H, 5.59; N, 8.78.

EXAMPLE 27

Preparation of N-(4-Formylphenyl)-N'-[3-(3-(3-pyridyl)propionic acid)]urea

To a cooled (4 °C) stirred solution of 1,1'-carbonyldiimidazole (3.24 g, 20.0 mmol) and imidazole (2.04 g, 30.0 mmol) in 65 mL of THF was added a solution of methyl 3-amino-3-(3-pyridyl)propionate (3.60 g,

20.0 mmol) in 25 mL of THF over 10 minutes. After stirring an additional 15 minutes, the cooling bath was removed. After 45 minutes, a solution of 4-aminobenzaldehyde (2.42 g, 20.0 mmol) in 100 mL of THF was rapidly added to the reaction solution. The reaction mixture was then heated to reflux for 24 h. The reaction mixture was concentrated and the residue purified by flash chromatography (silica gel, 6.5/93.5 $CH_3OH$/ $CH_2Cl_2$) to afford 5.01 g of crude product. The crude product was purified by flash chromatography (silica gel, 0.5/9.5 $CH_3OH$/ $CH_2Cl_2$) to afford 3.41 g (52%) of N-(4-formylphenyl)-N-[3-(methyl 3-(3-pyridyl)-propionate)]urea as yellow foam. A small sample was recrystallized from EtOAc for analysis, the remainder was used directly in the next reaction. $^1H$ NMR (DMSO-$d_6$) δ 9.79 (s, 1 H), 9.16 (s, 1 H), 8.59 (s, 1 H), 8.46 (d, J- 3.1 Hz, 1 H), 7.9-7.7 (m, 3 H), 7.65-7.5 (m, 2 H), 7.37 (dd, J= 4.8, 7.9 Hz, 1 H), 7.12 (s, J= 8.2 Hz, 1 H), 5.25-5.15 (m, 1 H), 3.56 (s, 3 H), 3.35-2.90 (m, 2 H); $^{13}C$ NMR (DMSO-$d_6$) δ 191.4, 171.3, 154.4, 148.6, 147.5, 245.5, 137.3, 135.0, 131.4, 130.7, 123.9, 118.0, 52.0, 48.4, 39.8. Anal. Calcd for $C_{17}H_{17}N_3O_4$: C, 62.38; H, 5.24; N, 12.84. Found: C, 62.01; H, 5.18; N, 12.65.

To a stirred suspension of N-(4-formylphenyl)-N'-[3-(methyl 3-(3-pyridyl)propionate)]urea in 90 mL of a 5/4 mixture of MeOH and water was added 7.60 mL of 1 N HCl followed by 15.2 mL of 1 N NaOH. After 26 h, the reaction mixture was partially concentrated to remove the MeOH, and diluted with 50 mL of water. The reaction solution was then washed with $CH_2Cl_2$ (3 x 50 mL ea.). The aqueous layer was decolorized with Norit A and filtered through celite and lyophilized. The residue was dissolved in 100 mL of ethanol and filtered to remove the insoluble NaCl. The filtrate was concentrated, the residue dissolved in 25 mL of water and lyophilized. The residue was purified by reverse phase chromatography and lyophilized to afford 1.92 g (76%) of the urea as a white powder: mp 200-205 °C decomp; $^1H$ NMR ($D_2O$) δ 9.71 (s, 1 H, CHO), 8.54 (s, 1 H), 8.42 (d, J= 4.9 Hz, 1 H), 7.9-7.7 (m, 3 H), 7.6-7.4 (m, 3 H), 5.14 (t, J= 7 Hz, 1 H), 2.8-2.65 (m, 2 H): $^{13}C$ NMR ($D_2O$) δ 194.7, 178.3, 155.8, 147.5, 146.7, 145.4, 138.5, 135.1, 131.6, 129.9, 124.2, 118.3, 50.1, 43.7. Anal. Calcd for $C_{16}H_{14}N_3O_4Na_1$-(0.16 $H_2O$): C, 56.83; H, 4.27; N, 12.43. Found: C, 56.80; H, 4.27; N, 12.43.

## EXAMPLE 28

### Preparation of N-(4-Cyanophenyl)-N'-[3-(5-phenylpentanoic acid)]urea sodium salt

A stirred suspension of 3-phenylpropionaldehyde (6.71 g, 50.0 mmol) and methyl (triphenylphosphoranylidene)-acetate (25.1 g, 75.0 mmol) in 150 mL of acetonitrile was refluxed for 1 h. The cooled reaction mixture was concentrated. The residue was slurried in 1/9 EtOAc/hexane (100 mL), and filtered. The filtrate was concentrated and purified by flash chromatography (1/9 EtOAc/hexane, silica gel) to afford 8.41 g (88%) of methyl 5-phenylpent-2-enoate as an oil: $^1H$ NMR ($CDCl_3$) δ 7.35-7.13 (m, 5 H), 7.00 (dt, 1 H, J= 6.8, 15.7 Hz), 5.84 (dt, 1 H, J= 1.5, 15.7 Hz), 3.70 (s, 3 H), 2.76 (t, 2 H, J= 7.5 Hz), 2.58-2.45 (m, 2 H); $^{13}C$ NMR ($CDCl_3$) δ 166.9, 148.3, 140.6, 128.4, 128.2, 126.1, 121.3, 51.3, 34.2, 33.8.

A solution of methyl trans-5-phenylpent-2-enoate (5.71 g, 30.0 mmol) and benzylamine (3.28 mL, 30.0 mmol) in 80 mL of methanol was stirred for 51 h. The reaction solution was concentrated and the residue purified to afford 2.64 g (46%) of starting olefin and 4.56 g (51%) of methyl N-benzyl-3-amino-5-phenylpentanoate as a clear oil: $^1H$ NMR ($CDCl_3$) δ 7.4-7.13 (m, 10 H), 3.78 (overlapping dd, 2 H, NCH2), 3.66 (s, 3 H, $CO_2CH_3$), 3.06 (m, 1 H), 2.68 (m, 2 H), 2.51 (d, 2 H, J= 6.1 Hz), 1.9-1.7 (m, 2 H), 1.53 (br s, 1 H); $^{13}C$ NMR ($CDCl_3$) δ 172.7, 142.0, 140.4, 128.3, 128.1, 126.9, 125.9, 53.7, 51.5, 50.8, 38.8, 36.1, 32.0. IR (KBr) $cm^{-1}$ 3080, 3040, 2950. 2860, 1730, 1500, 1460, 1440. Anal. Calcd for $C_{19}H_{23}N_1O_2$: C, 76.74; H, 7.80; N, 4.71. Found: C, 77.11; H, 7.93; N, 4.75.

To a solution of methyl N-benzyl-3-amino-5-phenylpentanoate in 50 mL of methanol was added 100 mg of 20% $Pd(OH)_2$. This suspension was treated with 50 psi of hydrogen in a Parr Type Shaker. After 15 h and 39 h, 100 mg of 20% $Pd(OH)_2$ was added. After 63 h, the reaction mixture was filtered through celite to remove the catalyst and the filtrate concentrated to afford 2.71 g (97%) of methyl 3-amino-5-phenylpentanoate as a clear oil: $^1H$ NMR ($CDCl_3$) δ 7.35-7.14 (m, 5 h, Ph), 3.68 (s, 3 H, $CO_2CH_3$), 3.28-3.15 (m, 1 H, CHN), 2.82-2.48 (m, 2H, $CH_2Ar$), 2.50 (dd, 1 H, J = 4 Hz, 15.7 Hz), 2.31 (dd, 1 H, J= 8.8 Hz, 15.7 Hz), 1.78-1.6 (m, 2 H), 1.47 (s, 2 H, $NH_2$); $^{13}C$ NMR ($CDCl_3$) δ 172.8, 141.6, 128.4, 128.3, 125.8, 51.5, 47.9, 42.5, 39.5, 32.4; IR (KBr) $cm^{-1}$ 3390, 3300, 3040, 2960, 2940, 2860, 1730, 1660, 1500, 1454, 1437. Anal. Calcd for $C_{12}H_{17}N_1O_2$: C, 69.54; H, 8.27; N, 6.76. Found: C, 69.98; H, 8.08; N, 6.30.

To a stirred solution of methyl 3-amino-5-phenylpentanoate (2.07 g, 9.99 mmol) in 35 mL of ethyl acetate was added 4-cyanophenyl isocyanate (1.44 g, 9.99 mmol). After 24 h, the reaction mixture was concentrated. The residue was slurried in 50 mL of ether and the slurry was filtered to afford after drying 3.01 g (86%) of the urea as an off-white powder: $^1H$ NMR (DMSO-$d_6$) δ 9.0 (s, 1 H), 7.65 (d, 2 H, J = 8.8 Hz), 7.57 (d, 2 H, J= 8.8 Hz), 7.22 ( m, 5 H, Ph), 6.47 (d, 1 H, J= 8.7 Hz, NH), 4.0 (m, 1 H), 3.57 (s, 3 H,

$CH_3$), 2.7-2.5 (m, 4 H), 1.77 (m, 2 H) contaminated with ethyl acetate; IR(KBr) $cm^{-1}$ 3340, 2240, 1730, 1680, 1600, 1550, 1520, 1320, 1240.

To a stirred suspension of the above urea (2.50 g, 7.11 mmol) in a mixture of 150 mL of methanol and 30 mL of water was added 28 mL of 1 N NaOH. The progress of the reaction was monitored by HPLC. After 44 h, the reaction mixture was partially concentrated to remove the methanol and the residue slurried in 100 mL of water. The resulting slurry was filtered to afford after drying in vacuo, 2.11 g (83%) of the product as a white solid: $^1H$ NMR (DMSO-$d_6$) δ 10.93 (br s, 1 H), 7.95 (br s, 1 H), 7.73 (d, 2 H, J = 8.4 Hz), 7.54 (d, 2 H, J= 8.4 Hz), 7.14 (m, 5 H), 3.9 (m, 1 H), 2.56 (m, 2 H), 2.23 (d, 2 H, J= 4.4 Hz), 1.7 (m, 2 H); IR(KBr) $cm^{-1}$ 3420, 3160, 3080, 3020, 2920, 2228, 1698, 1690, 1594, 1572, 1542, 15412, 1408, 1320, 1240, 1176. Anal. Calcd for $C_{19}H_{18}N_3O_3Na$-(1.32 $H_2O$): C, 59.56; H, 5.43; N, 10.97. Found: C, 59.26; H, 5.10; N, 11.10.

EXAMPLE 29

Preparation of (S)-N-(4-Cyanophenyl)-N'-[3-(3-(3-pyridyl)propionic acid)]

To a stirred solution of 3-pyridinecarboxaldehyde (21.4 g, 0.20 mol) in benzene (250 mL) was added (S)-1-phenylethylamine (24.2 g, 0.20 mol). The reaction mixture was refluxed for 2 h with a Dean-Stark trap. The reaction mixture was then allowed to cool to room temperature and concentrated. Purification of the residue by distillation afforded 40.8g (97 %) of N-[(S)-1-phenyethyl)]pyridine-3-carboxaldimine (1): B.p. 123 °C/0.25 Torr; $^1H$ NMR (300 MHz, $CDCl_3$) δ 1.59 (d, J = 6.6 Hz, 3H), 4.55 (q, J = 6.6 Hz, 1H), 7.21-7.43 (m, 6H), 8.14 (d, J = 7.9 Hz, 1H), 8.37 (s, 1H), 8.62 (d, J = 3.4 Hz, 1H), 8.7 (s, 1H). $^{13}C$ NMR (75.5 MHz, $CDCl_3$) δ 156.3, 151.3, 150.2, 144.6, 134.5, 131.7, 125.4, 126.9, 126.4, 123.4, 69.9, 24.7.

A stirred suspension of 32.7 g (5 equiv) of activated zinc dust in 300 mL of THF was heated to reflux under $N_2$. Several 0.1mL portions of methyl bromoacetate were added with vigorous stirring to initiate the reaction. When a green color appeared, 21.0 g (0.100 mol) of N-[(S)-1-phenyethyl)]pyridine-3-carboxaldimine in 100 mL of THF was added. Then 37.9 mL (4 equiv) of methyl bromoacetate was added dropwise over 45 min to the refluxing mixture. The mixture was refluxed for an additional 10 min, cooled to room temperature, diluted with 500 mL of THF, and the reaction quenched with 140 mL of 50% aqueous $K_2CO_3$. Rapid stirring for 45 min gave a suspension. The THF layer was decanted, and the residue was rinsed with THF. The combined THF layers were concentrated and the resulting crude oil dissolved in ethyl acetate. The reaction mixture was then washed with water and brine, dried ($MgSO_4$) and concentrated to afford 23.2 g (92 %) of a mixture of diastereomers (1:1) of the β-lactam (4S) and (4R)[(S)-N-phenyethyl]-3-amino-3-(3-pyridyl)propionate and β-(phenylethylamine)-(3-pyridyl)methylpropionate.

The product obtained from the above reaction was dissolved in 200 mL of 6N HCl. The reaction mixture was refluxed for 15 min, cooled to room temperature, partially concentrated and the pH adjusted to 4-5 with basic resin. The reaction mixture was filtered, and concentrated. The residue was dissolved in methanol, dried over $MgSO_4$, filtered and concentrated to afford an oil consisting of a mixture of the diastereomers, N-(S)-phenyethyl-3-(R,S)-amino-3-(3-pyridyl)propionic acid.

To the residue (24.8 g) obtained by the above procedure was added 19.8 g (0.24 mol) of benzyl alcohol in 200 mL of methylene chloride and 1.0 g of DMAP. The reaction mixture was cooled to 0 °C and 37.7 g (0.18 mol) of DCC in 100 mL of methylene chloride was added. The mixture was allowed to warm to room temperature and stirred an additional 12 h. The reaction mixture was then filtered to remove the DCU and washed with water, brine, and dried ($MgSO_4$). After silica chromatography (elution with 1:1 hexane-ethyl acetate), 3.91 g (12%) of benzyl N-[(S)-phenyethyl]-3-(S)-amino-3-(3-pyridyl)propionate was isolated from the mixture of diastereomers as an oil. $R_f$ = 0.32 (ethyl acetate); $^1H$ NMR (300 MHz, $CDCl_3$) δ 1.25 (d, J = 6.7 Hz, 3H), 2.20 (bs, 1H), 2.65 (ddd, J = 15.4, 9.0, 5.1 Hz, 2H), 3.40 (q, J = 6.7 Hz, 1H), 3.80 (dd, J = 8.9, 5.1 Hz, 1H), 5.10 (dd, J = 27.0, 12.2 Hz, 2H), 7.10 (d, J = 6.4 Hz, 2H), 7.23-7.47 (m, 9H), 7.56 (d, J = 7.8 Hz, 1H), 8.40 (s, 1H), 8.52 (d, J = 4.8 Hz, 1H); $^{13}C$ NMR (75.5 MHz, $CDCl_3$) δ 170.9, 149.2, 149.0, 144.5, 137.7, 135.5, 134.7, 128.5, 125.3, 127.0, 126.5, 123.5, 66.4, 55.0, 54.2, 42.9, 24.9.

To a stirred suspension of 3.0 g of the same amino ester and an equal weight of 10% Pd/C in dry methanol (50 mL), was added anhydrous ammonium formate (5.2 g, 83 mmol) in a single portion under nitrogen. The resulting reaction mixture was stirred at reflux for 6 h and then the catalyst was removed by filtration through a celite pad. The reaction mixture was concentrated and refluxed in methanol (30 mL) while 30 mL of ethyl acetate was slowly added over 15 min. The slurry was allowed to cool to room temperature, and filtered to afford 457 mg of the β-amino acid, (S)-3-amino-3-(3-pyridyl)propionic acid. The residue from the filtrate was resubmitted to the above conditions to yield another 210 mg of the β-amino acid, (S)-3-amino-3-(3-pyridyl)propionic acid. The total yield was 667 mg (48%) of the amino acid. $^1H$ NMR (300 MHz, $D_2O$) δ 2.98 (dq, J = 18.2, 6.9 Hz, 2H), 4.73 (t, J = 7.3 Hz, 1H), 7.52 (dd, J = 17.5, 5.0 Hz, 1H),

7.96 (d, J = 8.0 Hz, 1H), 8.55 (d, J = 20 Hz, 1H), 8.59 (s, 1H); $^{13}$C NMR (75.5 MHz, $CDCl_3$) δ 176.6, 149.5, 147.7, 136.3, 132.6, 124.9, 50.5, 40.0.

To a solution of sodium hydroxide (120 mg, 3 mmol) and 498 mg (3.4 mmol) of (S)-3-amino-3-(3-pyridyl)propionic acid in methanol (45 mL) was rapidly added a solution of p-cyanophenyl isocyanate in methyl acetate (65 mL). The temperature of the reaction mixture dropped 2-5 °C after the addition. The reaction mixture was then stirred for 15 min and concentrated. The residue was dissolved in methanol (5 mL) and ethyl acetate (5 mL) and refluxed until the solution becomes turbid (2-5 min). To this mixture was added ethyl acetate (45 mL) slowly, and the heating was stopped halfway through the addition. The mixture was allowed to cool slowly to 45 °C, at which time the solid was filtered off. The solid was washed with ethyl acetate (2 X 2.5 mL) and dried to afford 900 mg (90%) of the product as a white solid. $[\alpha]^{26}$ = 59 5° (c 5.12, $H_2O$). $^1$H NMR (300 MHz, $D_2O$) δ 2.69 (dd, J = 7.2, 1.8 Hz, 2H), 5.09 (t, J = 6.4 Hz, 1H), 7.26 (d, J = 8.8 Hz, 2H), 7.39 (dd, J = 7.9, 4.9 Hz, 1H), 7.45 (d, J = 8.8 Hz, 2H), 7.81 (dt, J = 8.0, 1.5 Hz, 1H), 8.36 (dd, J = 4.9, 1.2 Hz, 1H), 8.49 (d, J = 1.8 Hz, 1H). $^{13}$C NMR (75.5 MHz, $D_2O$) δ 178.5, 156.0, 147.6, 146.8, 143.3, 138.6, 135.2, 133.4, 124.3, 120.1, 118.8, 103.8, 50.2, 43.8. Anal. Calcd for $C_{16}H_{13}N_4NaO_3$-$6H_2O$ (343.10) δ C 56.01, H 4.17, N 16.03; found: C 56.10 , H 4.08, N 16.14.

EXAMPLE 30

Conversion of (S)-N-(4-Cyanophenyl)-N'-[3-(3-(3-pyridyl)propionic acid)]urea to (S)-N-(4-Carbamoylphenyl)-N'-[3-(3-(3-pyridyl) propionic acid urea sodium salt

Hydrogen peroxide (30%, 0.3 mL, 2.64 mmol) was added to a stirred suspension of (S)-N-(4-Cyanophenyl)-N'-[3-(3-(3-pyridyl)propionic acid)]urea (0.250 g, 0.753 mmol) in ethanol (1 mL), water (1 mL) and sodium hydroxide (6N, 0.2 mL, 1.20 mmol). The reaction mixture was stirred for 25 min at room temperature until the contents of the flask became clear and the evolution of gas (oxygen) stopped. Sodium bisulfite (0.2 g) was added to the reaction mixture to destroy excess hydrogen peroxide. The reaction mixture was concentrated in vacuo at room temperature and then chromatrographed (PRP-1 column HPLC, 2% acetonitrile in water as the eluant). Pure fractions were combined and lyophilized to afford 0.20 g (76%) of the desired product as a white crystalline powder. $^1$H NMR ($D_2O$) d 2.72 (d, 2H, J=7.0 Hz), 5.13 (t, 1H, J=7.0 Hz), 7.37 and 7.73 (AB quartet, 4H, J=7.1 Hz), 7.42-7.48 (m, 1H), 7.88 (d, 1H, J=7.7 Hz), 8.43 (m 1H), 8.53 (m, 1H).

EXAMPLE 31

Preparation of (S)-N-(4-Cyanophenyl)-N'-[3-(3-phenylpropionic acid)]urea

(S)-3-amino-3-phenylpropionic acid hydrochloride was separated from commercially available 3-amino-3-phenylpropionic acid hydrochloride (Aldrich) by the method of Fisher, Scheibler, and Groh as it appears in "Chem. Ber.", Vol. 43 pages 2020-3-(1910). The compound, 1.08 grams, was a single peak by HPLC (chiral); $[\alpha]D_{20}$ + 2.36, 3.0% in MeOH; lit. [α]24D + 3.3o, 2.95% in MeOH. Anal. Calcd for $C_9H_{11}NO_2$-HCl-$(H_2O)_{0.11}$: C, 53.08; H, 6.05; N, 6.88. Found: C, 53.06; H,6.04; N, 6.82.

To a stirred suspension of (S)-3-amino-3-phenylpropionic acid hydrochloride (1.00 g, 6.05 mmol) and 4-cyanophenyl isocyanate (1.0 g, 6.9 mmol) in 50 mL of acetonitrile was added 13 mmol of 1 N NaOH. The clear solution which immediately formed was stirred overnight before the solvents were removed at reduced pressure. The residue was dissolved in 100 mL of water and washed with ethyl acetate (2 x 50 mL). The aqueous layer was acidified to a pH of 2 with concentrated HC1 to produce a gummy solid. The gum yielded, after thorough drying in a vacuum oven, 1.20 grams (64%) of the desired product, as a brittle white solid. The product showed one peak on HPLC using a Daicel Chiral pak WH column; IR (KBr) $cm^{-1}$ 3360, 2220, 1710, 1670, 1590, 1540, 1410, 1320, 1240, 1180; 1H NMR (DMSO-$d_6$) d 9.2 (s, 1H), 7.7 (d, 2H, J=8.7Hz), 7.6 (d, 2H, J=8.7Hz), 7.3 (m, 5H), 7.1 (d, 1H, J=8.7Hz), 5.2 (q, 1H), 2,8 (m, 2H); $[\alpha]D_{21}$-3.45°, 5.0% in MeOH. Anal. Calcd for $C_{17}H_{15}N_3O_3.(H2O)_{0.5}$: C, 64.29; H, 5.05; N, 13.23. Found C, 64.28; H, 5.08; N, 12.96.

EXAMPLE 32

Preparation of N-[5-(2-Cyanopyridyl)]-N'-[3-(3-(3-pyridyl)propionic acid]urea Sodium salt

A solution of 2-cyano-5-pyridylcarbonylazide (4.05 g, 23.3 mmol) in 100 mL of dried toluene was heated at 80°C for three hours. To this cooled solution was added 4.23 g (22.4 mmol) of 3-amino-3-phenylpropionic acid sodium salt and the slurry stirred overnight at room temperature. The solvent was removed at reduced pressure and the residue chromatographed using a water mobile phase on a PRP-1 preparative column. The desired fractions were combined and lyophilized to give 1.4 grams (18%) of a white fluffy powder: IR (KBr) $cm^{-1}$ 3400, 2230, 1700, 1580, 1560, 1400, 1240; $^1H$ NMR ($D_2O$) $\delta$ 8.5 (m, 3H), 7.9 (m, 2H), 7.7 (d, 1H, J=8.7Hz), 7.45 (m, $_1$H), 5.2 (m, 1H), 2.8 (m, 2H): $^{13}C$ NMR ($D_2O$) $\delta$ 181.2, 158.5, 150.5, 149.6, 143.9, 142.6, 141.2, 138.0, 132.8, 128.6, 127.1, 127.0, 120.4, 53.1, 46.5.

EXAMPLE 33

Preparation of N-[5-(2-Cyanopyridyl)]-N'-[3-(3-phenylpropionic acid)]urea

To a solution of 3-amino-3-phenylpropionic acid (2.00 g, 12.0 mmol) in 24 mL 0.5 N NaOH was added a solution of 2-cyano-5-pyridyl isocyanate (2.03 g, 13.9 mmol) in 20 mL of acetonitrile:acetone. The reaction mixture was stirred overnight and then the solvents removed at reduced pressure on a RotoVac. The residue ws dissolved in 150 of equal parts of water and dichloromethane. The aqueous layer was extraced with dichloromethane (2 x 50 mL) and acidified to a pH of 2-3 with dilute HC1. The gummy precipitate was stirred overnight and the desired product isolated by filtration to yield 1.4 g (37%) of a white powder: mp 103-107°C; IR (KBr) $cm^{-1}$ 3350, 2233, 1700, 1680, 1540, 1235; $^1H$ NMR (DMSO-$d_6$) $\delta$ 9.4 (s, 1H), 8.6 (m, 1H), 8.1 (m, 1H), 7.9 (m, 1H), 7.2-7.4 (m, 6H), 5.2 (q, 1H), 2.8 (m, 2H); $^{13}C$ NMR (DMSO-d6) $\delta$ 172.3, 154.3, 143.5, 142.3, 142.0, 131.2, 130.1, 128.9, 127.9, 125.4, 119.6, 53.2, 51.8.

EXAMPLE 34

Preparation of N-(6-Indazolyl)-N'-[3-(3-phenylpropionic acid)urea)]

To a stirred solution of 1,1'-carbonyldiimidazole (1.82 g, 11.2 mmol) and imidazole (1.14 g, 16.8 mmol) in 30 mL of THF at RT was added a solution of methyl 3-phenylpropionate (2.00 g, 11.2 mmol) in 10 mL of THF over 20 minutes. Then, a suspension of 6-aminoindazole (1.49 g, 11.2 mmol) in 20 mL of THF was rapidly added. After 1 h, the reaction mixture was refluxed for 16 h. The reaction mixture was then concentrated. The residue was purified by flash chromatography (silica gel, 4/96 methanol/dichloromethane) to yield a slightly impure sample of N-(6-indazolyl)-N'-[3-(methyl 3-phenylpropionate)]urea. This sample was purified by flash chromatography (silica gel, 16/84 ethyl acetate/dichloromethane) to afford 0.86 g (23%) of the desired ester which was used in the next reaction.

To a stirred solution of N-(6-Indazolyl)-N'-[3-(methyl 3-phenylpropionate)]urea(0.800 g, 2.36 mmol) in 8 mL of methanol was added 2.36 mL of 1 N NaOH(aq). After 71 h, the reaction solution was partially concentrated to remove the methanol and diluted to a volume of 25 mL with water. The resulting slurry was washed with ethyl acetate (2 x 25 mL ea.). The aqueous layer was partially concentrated to remove traces of ethyl acetate and then acidified with 3.0 mL of 1 N HCl followed by the addition of 0.5 g of NaOH. A gum formed which solidified on stirring. The slurry was filtered and the solid dried to afford 0.56 g (73%) of the urea: $^1H$ NMR (DMSO-$d_6$) $\delta$ 12.35 (br s, 1 H), 8.68 (d, 1 H, J= 8.9 Hz), 2.68 (dd, 1 H); $^{13}C$ NMR (DMSO-$d_6$) $\delta$ 172.1, 151.9, 150.4, 142.4, 141.1, 137.7, 128.3, 121.5, 116.6, 113.4, 95.3, 50.4.

EXAMPLE 35

N-[5-(2-Carbamoylpyridyl)]-N'-[3-(3-(3-pyridyl)propionic acid)]urea Sodium Salt

To a stirred solution of N-[5-(2-cyanopyridyl)]-N'-[3-(3-(3-pyridyl)propionic acid)]urea sodium salt (108 mg, 0.32 mmol) in 3 mL of 1:1 ethanol/water were added 0.1 mL of 6 N NaOH (0.6 mmol) and 0.15 mL of 30% hydrogen peroxide. The reaction was stirred for thirty minutes at room temperature at which time 0.3 g of sodium bisulfite was added to quench the reaction. The solvents were removed at reduced pressure and the residue chromatographed on a PRP-1 preparative chromatography column. The desired fractions were combined and lyophilized to give 30 mg of the desired urea as a white solid; IR (KBr) $cm^{-1}$ 3400, 1680,

1580, 1550, 1400, 1240: $^1$H NMR ($D_2O$) δ 8.4 (s, 1H), 8.3 (s, 2H), 7.8-7.6 (m, 3H), 7.3 (m, 1H), 5.6 (t, 1H, J=7.3Hz), 2.6 (d, 2H, J=7.3Hz); $^{13}$C NMR ($D_2O$) δ 182.2, 173.0, 159.8, 151.3, 150.5, 145.9, 143.2, 142.7, 142.3, 139.0, 130.2, 128.1, 127.1, 54.0, 47.6.

EXAMPLE 36

N-[5-(2-Carbamoylpyridyl)]-N'-[3-(3-phenylpropionic acid)]urea Sodium Salt

To a stirred suspension of N-[5-(2-cyanopyridyl)]-N'[methyl 3-(3-phenylpropionate)]urea (108. mg, 0.33 mmol) in 3 mL of 1:1 ethanol/water were added 0.15 mL of 6 N NaOH (0.90 mmol) and 0.15 mL of 30% hydrogen peroxide. The reaction was stirred for 30 minutes at room temperature at which time 0.3 g of sodium bisulfite was added to quench the reaction. The solvents were removed at reduced presssure and the residue chromatographed on a PRP-1 preparative chromatography column. The desired fractions were combined and lyophilized to give 90 mg (78%) of the desire urea as a fluffy white powder; IR (KBr) cm$^{-1}$ 3320, 1680, 1580, 1560, 1560, 1410, 1240: $^1$H NMR (DMSO-$d_6$) δ 11.6 (s, 1H), 9.25 (s, 1H), 8.75 (s, 1H), 8.1 (d, 1H, J=9Hz), 7.9 (s, 1H), 7.8 (d, 1H, J=9Hz), 7.4-7.1 (m, 6H), 5.1 (m, 1H), 2.4 (m, 2H); $^{13}$C NMR (DMSO-$d_6$) δ 175.5, 166.4, 155.2, 146.1, 141.7, 141.1, 137.9, 128.0, 126.1, 123.6, 122.1, 52.24, 46.0.

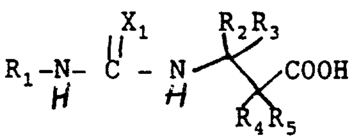

| | $R_1$ | $X_1$ | $R_2$ | $R_3$ | $R_4$ | $R_5$ |
|---|---|---|---|---|---|---|
| Ex. 1 | 4-Ethoxycarbonylphenyl | O | 3-Phenyl | H | H | H |
| Ex. 2 | 4-Acetylphenyl | O | 3-Phenyl | H | H | H |
| Ex. 3 | 4-Bromophenyl | O | 3-Phenyl | H | H | H |
| Ex. 4 | 4-Cyanophenyl | O | 3-Phenyl | H | H | H |
| Ex. 5 | 4-Cyanophenyl | O | 3-Pyridyl | H | H | H |
| Ex. 6 | 4-Nitrophenyl | O | 3-Phenyl | H | H | H |
| Ex. 7 | 4-Carbomoylphenyl | O | 3-Phenyl | H | H | H |
| Ex. 8 | 4-Sulfamylphenyl | O | 3-Phenyl | H | H | H |
| Ex. 9 | 4-Carbomethoxylphenyl | O | 3-Phenyl | H | H | H |
| Ex. 10 | 4-Carboethoxyphenyl | O | 3-Pyridyl | H | H | H |
| Ex. 11 | 4-Carbamoylphenyl | O | 3-Pyridyl | H | H | H |
| Ex. 12 | 4-Carboxyphenyl | O | 3-Pyridyl | H | H | H |
| Ex. 13 | 4-Iodophenyl | O | 3-Phenyl | H | H | H |
| Ex. 14 | 4-Chlororphenyl | O | 3-Phenyl | H | H | H |
| Ex. 15 | 3-Chlorophenyl | O | 3-Phenyl | H | H | H |
| Ex. 16 | 4-Methylphenyl | O | 3-Phenyl | H | H | H |
| Ex. 17 | 4-Trifluorophenyl | O | 3-Phenyl | H | H | H |
| Ex. 18 | 4-Cyanophenyl | O | 4-Methoxyphenyl | H | H | H |

| | | | | | | |
|---|---|---|---|---|---|---|
| Ex. 19 | 4-Cyanophenyl | 0 | 3,4-Dimethoxy-phenyl | H | H | H |
| Ex. 20 | 4-Cyanophenyl | S | 3-Phenyl | H | H | H |
| Ex. 21 | 4-Methoxycarbonylphenyl | S | 3-Phenyl | H | H | H |
| Ex. 22 | 4-Cyanophenyl | 0 | 4-Pyridyl | H | H | H |
| Ex. 23 | 4-Carboxyphenyl | 0 | 3-Phenyl | H | H | H |
| Ex. 24 | Phenyl | 0 | 3-Phenyl | H | H | H |
| Ex. 25 | 4-Formylphenyl | 0 | 3-Phenyl | H | H | H |
| Ex. 26 | 4-Hydroxyphenyl | 0 | 3-Phenyl | H | H | H |
| Ex. 27 | 4-Formylphenyl | 0 | 3-Pyridyl | H | H | H |
| Ex. 28 | 4-Cyanophenyl | 0 | Phenyethyl | H | H | H |
| Ex. 29 | 4-Cyanophenyl | 0 | (S)-3 Pyridyl | H | H | H |
| Ex. 30 | 4-Carbamoyl | 0 | (S)-3 Pyridyl | H | H | H |
| Ex. 31 | 4-Cyanophenyl | 0 | (S)-3-Phenyl | H | H | H |
| Ex. 32 | 5-(2-Cyanopyridyl) | 0 | 3-Pyridyl | H | H | H |
| Ex. 33 | 5-(2-Cyanopyridyl) | 0 | 3-Phenyl | H | H | H |
| Ex. 34 | 6-Indazolyl | 0 | 3-Phenyl | H | H | H |
| Ex. 35 | 5-(2-Carbamoylpyridyl) | 0 | 3-Phenyl | H | H | H |
| Ex. 36 | 5-(2-Carbamoylpyridyl) | 0 | 3-Phenyl | H | H | H |

## Claims

**1.** A compound corresponding to the formula

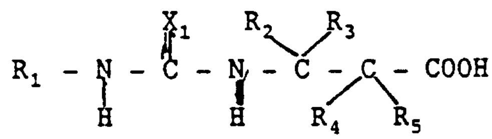

wherein $X_1$ is O or S, wherein $R_1$ is optionally substituted phenyl, said phenyl corresponding to

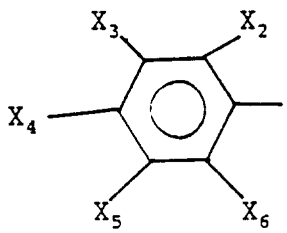

wherein $X_2$, $X_3$, $X_4$, $X_5$ and $X_5$ are the same or different and are selected from the group consisting of:

H,
$CF_3$,
$CF_2CF_3$,
$CH_2CF_3$,
$C_1$-$C_4$ alkyl,
$CH=NOCH_3$,
$CH=NOH$,
CHO,
$CH_2OCH_3$,
$CH_2OH$,
CN,
$COCF_3$,
$COC_1$-$C_3$ alkyl,
$CONH_2$,
$CONHC_1$-$C_3$ alkyl,
$CON(C_1$-$C_3$ alkyl$)_2$,
$COOC_1$-$C_3$ alkyl,
COOH,
$NH_2$,
$NHC_1$-$C_3$ alkyl,
$N(C_1$-$C_3$ alkyl$)_2$,
NHCHO,
Cl, with the proviso that $X_3$ and $X_5$ may not both be Cl,
Br,
I,
F,
$NHCOCH_3$,
$NHCONH_2$,
$NHSO_2CH_3$,
$C_1$-$C_3$ alkyl COOH,
$NO_2$,
$OC_1$-$C_3$ alkyl, with the proviso that $X_4$ may not be $OCH_2CH_3$
$OCOCH_3$,
OH,
$SC_1$-$C_3$ alkyl,

$SOC_1$-$C_3$ alkyl,
$SO_2C_1$-$C_3$ alkyl,
$SO_2NH_2$,
$SO_2NHC_1$-$C_3$ alkyl,
$SO_2NC(C_1$-$C_3$ alkyl$)_2$,
$SO_3H$,
and where substituents at any two of $X_2$, $X_3$, $X_4$, $X_5$ or $X_5$ form a fused ring,

or wherein $R_1$ is selected from the group consisting of optionally substituted pyridyl, optionally substituted pyrimidyl, 2-indanyl, or 6-indazolyl, and

wherein $R_3$, $R_4$, and $R_5$ are hydrogen and wherein $R_2$ is phenyl optionally substituted by one or two alkoxy groups or a nitro group, pyridyl or 2-phenylethyl.

**2.** The compound of claim 1 wherein $R_1$ is an optionally substituted phenyl wherein $X_4$ is selected from the group consisting of CN, $NO_2$, $CO_2CH_3$, $CONH_2$, HCO, $SO_2NH_2$, $CH_3SO_2$, and $CO_2C_2H_5$.

**3.** The compound of claim 1 wherein $R_1$ is an optionally substituted pyridyl.

**4.** The compound of claim 1 wherein $R_1$ is an optionally substituted pyrimidyl.

**5.** The compound of claim 1 wherein $R_2$ is selected from the group consisting of phenyl, 2-pyridyl, 3-pyridyl, or 4-pyridyl.

**6.** The compound of claim 1 wherein $X_1$ is O.

**7.** The compound of claim 1 having the formula:

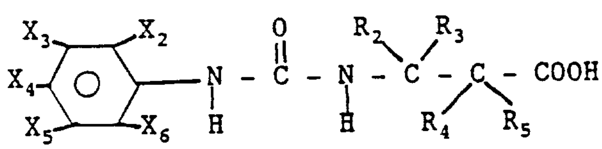

**8.** The compound of claim 7 wherein $X_2$, $X_3$, $X_5$ and $X_5$ are H and $X_4$ is selected from the group consisting of CN, $NO_2$, $CO_2C_2H_5$, $CO_2CH_3$, $CONH_2$, Cl, Br, F, I, HCO, $CH_3CO$, $SO_2NH_2$ and $CH_3SO_2$.

**9.** The compound of claim 7 wherein $R_2$ is selected from the group consisting of phenyl, 2-pyridyl, 3-pyridyl, 4-pyridyl.

**10.** The compound of claim 8 wherein $X_4$ is selected from the group consisting of CN, $NO_2$, $CONH_2$, CHO, $CO_2CH_3$ and $CO_2C_2H_5$.

**11.** The compound of claim 10 wherein $R_2$ is selected from the group consisting of 2-pyridyl, 3-pyridyl, 4-pyridyl and phenyl.

**12.** The compound of claim 10 wherein $X_2$, $X_3$, $X_5$ and $X_5$ are H, $X_4$ is selected from the group consisting of CN, $NO_2$, $CONH_2$, HCO, $CO_2C_2H_5$, $CO_2CH_3$, Cl, Br, F, I, $CH_3CO$, $CH_3SO_2$, and $SO_2NH_2$, $R_3$, $R_4$ and $R_5$ are H, and $R_2$ is selected from the group consisting of phenyl, 2-pyridyl, 3-pyridyl, 4-pyridyl.

**13.** The compound of claim 12 wherein $X_4$ is CN and $R_2$ is 3-pyridyl.

**14.** The compound of claim 12 wherein $X_4$ is CN and $R_2$ is phenyl.

**15.** The compound of claim 12 wherein $X_4$ is CN and $R_2$ is 4-pyridyl.

**16.** The compound of claim 12 wherein $X_4$ is $NO_2$ and $R_2$ is phenyl.

**17.** The compound of claim 12 wherein $X_4$ is $CO_2C_2H_5$ and $R_2$ is phenyl.

**18.** The compound of claim 4 wherein $R_3$, $R_4$ and $R_5$ are H and $R_2$ is selected from the group consisting of phenyl, 2-pyridyl, 3-pyridyl, 4-pyridyl, naphthyl.

**19.** The compound of claim 12 wherein $X_4$ is $CONH_2$ and $R_2$ is 3-pyridyl.

**20.** The compound of claim 13 wherein $X_4$ is CHO and $R_2$ is 3-pyridyl.

**21.** The compound of claim 12 wherein $X_4$ is $CONH_2$ and $R_2$ is phenyl.

**22.** The compound of claim 12 wherein $X_4$ is CHO and $R_2$ is phenyl.

**23.** The compound of claim 12 wherein $X_4$ is $CONH_2$ and $R_2$ is 4-pyridyl.

**24.** The compound of claim 12 wherein $X_4$ is CHO and $R_2$ is 4-pyridyl.

**25.** The compound of claim 19 wherein $R_1$ is 5-(2-cyanopyridyl) and $R_2$ is 3-pyridyl.

**26.** The compound of claim 19 wherein $R_1$ is 5-(2-cyanopyridyl) and $R_2$ is phenyl.

**27.** The compound of claim 1 wherein the compound is selected from the group of physiologically acceptable salts comprising hydrochloride, phosphate, citrate, sulfate, bisulfate, sodium, potassium, ammonium, calcium, malate, tosylate, benzoate and magnesium salts.

**28.** A process for sweetening edible products comprising foods, beverages, confections, chewing gums, pharmaceuticals, veterinary preparations and toilet, cosmetic and hygiene products characterized in that an effective sweetening amount of a compound of claim 1 is added to said edible products.

**29.** Edible products sweetened according to the process of claim 28.

**30.** Sweetening compositions characterized in that said compositions comprise an effective sweetening amount of a compound of claim 1 and a physiologically acceptable carrier therefor.

**31.** The sweetening compositions of claim 30 wherein the carrier is a bulking agent.

**32.** The sweetening compositions of claim 30 wherein the carrier is selected from the group consisting of water, polymeric dextrose, starch and modified starches, maltodextrins, cellulose, methylcellulose, cellobiitol, carboxymethylcellulose, maltitol, hydroxypropylcellulose, hemicelluloses, microcrystalline cellulose, other cellulose derivatives, sodium alginate, pectins and other gums, lactose, maltose, glucose, leucine, glycerol, mannitol, sorbitol, sodium bicarbonate, and phosphoric, citric, tartaric, fumaric, benzoic, sorbic, and propionic acids and their sodium, potassium and calcium salts and mixtures of any of the above.

**33.** A sweetening composition comprising:
(a) a first sweetening agent comprising a compound of claim 1; and
(b) a second sweetening agent which is not a compound of claim 1.

**34.** The sweetening composition of claim 33 further comprising a bulking agent.

**35.** The sweetening composition of claim 33 wherein said second sweetening agent is selected from the group consisting of sucrose, corn syrups, fructose, aspartame, alitame, neohesperidin dihydrochalcone, high fructose corn syrup, hydrogenated isomaltulose, stevioside type sweeteners, L-sugars, lactitol, neosugar, glycyrrhizin, xylitol, acesulfam-K, sodium saccharin, potassium saccharin, calcium saccharin, cyclamic acid and the sodium, potassium, and calcium salts thereof, sucralose, monellin, thaumatin and mixtures thereof.

**36.** A process comprising

(a) reacting a compound of the formula:

$R_1NCX_1$

with a compound of the formula:

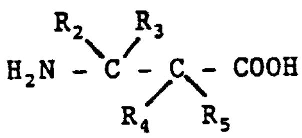

wherein $X_1$ is O or S, wherein the substituents $R_1$-$R_5$ are defined according to claim 1; and
(b) recovering the urea compound formed in step (a) above.

**37.** The process of claim 36 wherein $R_1$ is an optionally substituted phenyl, optionally substituted pyridyl, or optionally substituted pyrimidyl.

**38.** The process of claim 36 wherein $R_1$ is an optionally substituted phenyl wherein $X_2$, $X_3$, $X_5$ and $X_5$ are H, and $X_4$ is selected from the group consisting of CN, $NO_2$, $CO_2C_2H_5$, $CO_2CH_3$, $CONH_2$, Cl, Br, F, I, HCO, $CH_3CO$, $SO_2NH_2$ and $CH_3SO_2$, and $X_1$ is O.

**39.** The process of claim 36 wherein $R_2$
is selected from the group consisting of phenyl, 2-pyridyl, 3-pyridyl, 4-pyridyl.

**40.** The process of claim 36 wherein $X_4$ is selected from the group consisting of CN, $NO_2$, $CONH_2$, CHO, $CO_2CH_3$, and $CO_2C_2H_5$.

**41.** The process of claim 36 wherein $R_2$ is selected from the group consisting of 2-pyridyl, 3-pyridyl, 4-pyridyl and phenyl.

**42.** The process of claim 36 wherein $R_1$ is an optionally substituted pyridyl.

**43.** The process of claim 36 wherein $R_1$ is an optionally substituted pyrimidyl.

**44.** The process of claim 36 wherein step (a) is carried out in the presence of a base.

**45.** The process of claim 36 wherein step (a) is carried out in the presence of a solvent.

**46.** The process of claim 45 wherein said solvent is acetonitrile.

**47.** The process of claim 45 wherein said solvent is a mixture of acetonitrile and water.

**48.** A sweet foodstuff including one or more compounds of Claim 1 as the sweetening agent.

**49.** An edible composition comprising
(a) a foodstuff; and
(b) one or more sweetening agents selected from the group consisting of the compounds of Claim 1.

**50.** A process comprising
(a) reacting a compound of the formula

$R_1NH_2$

with a compound of the formula

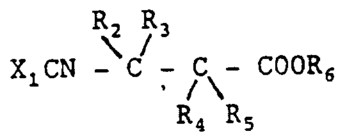

wherein $X_1$ is O or S, wherein the substituents $R_1$-$R_5$ are defined according to Claim 1; and wherein $R_6$ is methyl, ethyl, propyl, or butyl, and

(b) hydrolyzing the resulting compound; and

(c) recovering the isolated desired urea compound or salt thereof formed in step (a).

**51.** The edible composition of claim 49 further comprising a sweetening agent selected from the group consisting of sucrose, corn syrups, fructose, aspartame, alitame, neohesperidin dihydrochalcone, high fructose corn syrup, hydrogenated isomaltulose, stevioside type sweeteners, L-sugars, lactitol, neosugar glycyrrhizin, xylitol, acesulfam-K, sodium saccharin, potassium saccharin, calcium saccharin, cyclamic acid and the sodium, potassium, and calcium salts thereof, sucralose, monellin, thaumatin and mixtures thereof.

**52.** The edible composition of claim 49 comprising a beverage.

**53.** The edible composition of claim 49 comprising a confection.

**54.** A compound for use in preparing the compositions of claim 1 corresponding to the formula

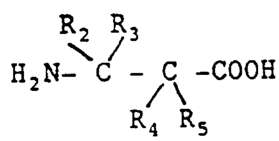

or salts thereof wherein $R_2$, $R_3$, $R_4$, and $R_5$ are defined according to Claim 1.

**55.** A process for producing a first urea or thiourea of the formula

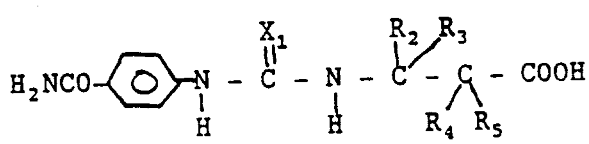

and all salts thereof from a second urea or thiourea of the formula

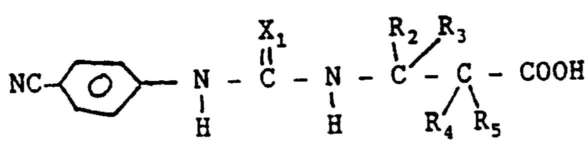

and all salts thereof wherein $X_1$ is O or S, and wherein

$R_2$, $R_3$, $R_4$ and $R_5$ are defined according to Claim 1 comprising

enantiomers and physiologically acceptable salts thereof, said process

comprising the step of:

reacting said second urea or thiourea with alkaline hydrogen peroxide to produce said first urea or thiourea.

**56.** A process for obtaining one isomer of a first compound of the formula:

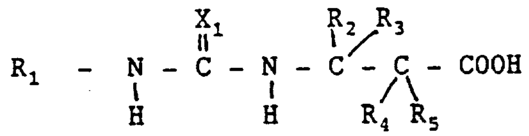

and all salts thereof wherein $X_1$ is O or S, $R_1$-$R_5$ are defined according to Claim 1,
comprising the steps of:
reacting an aldehyde with an amine to produce a Schiff base;
reacting said Schiff base with a methyl haloacetate and a metal to produce a diastereomeric mixture of a β-lactam;
hydrolyzing said β-lactam to produce a diastereomeric mixture of a first β-amino acid;
esterifying said first β-amino acid;
isolating one isomer of the ester of said diastereomeric mixture of said first β-amino acid;
hydrogenolyzing said ester to produce one stereoisomer of a second β-amino acid;
reacting said stereoisomer of the second amino acid with an isocyanate or isothiocyanate to produce said first compound.

**57.** The process of claim 56 wherein said metal is zinc.

**58.** The process of claim 56 wherein said second β-amino acid is produced by reaction of said first β-amino acid with palladium and carbon.

**59.** A process for obtaining one isomer of a first compound of the formula:

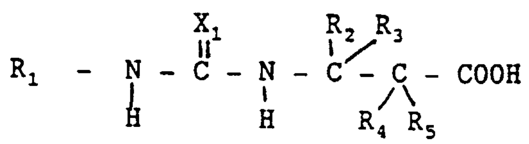

and all salts thereof wherein $X_1$ is O or S, $R_1$ -$R_5$ are defined according to Claim 1,
comprising the steps of:
reacting an aldehyde with an amine to produce a Schiff base;
reacting said Schiff base with methyl haloacetate and a metal to produce a diastereomeric mixture of a β-lactam;
isolating one diastereomer of said β-lactam;
hydrolyzing said isomer of said β-lactam to produce one stereoisomer of first β-amino acid;
hydrogenolyzing said stereoisomer of first β-amino acid to produce one stereoisomer of said second β-amino acid;
reacting said stereoisomer of said second β-amino acid with an isocyanate or isothiocyanate to produce said first compound.

## Patentansprüche

**1.** Eine Verbindung entsprechend der Formel

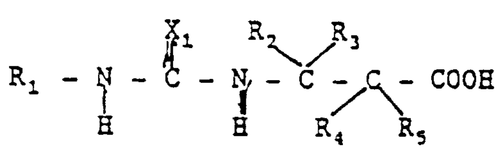

worin $X_1$ O oder S ist, $R_1$ wahlweise substituiertes Phenyl ist, wobei dieses Phenyl der Formel

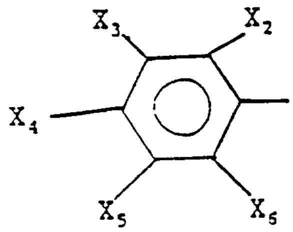

entspricht, worin $X_2$, $X_3$, $X_4$, $X_5$ und $X_5$ gleich oder verschieden sein können und ausgewählt sind aus der Gruppe bestehend aus:

H,
$CF_3$,
$CF_2CF_3$,
$CH_2CF_3$,
$C_1$-$C_4$-Alkyl,
$CH=NOCH_3$,
$CH=NOH$,
CHO,
$CH_2OCH_3$,
$Ch_2OH$,
CN,
$COCF_3$,
$COC_1$-$C_3$-Alkyl,
$CONH_2$,
$CONHC_1$-$C_3$-Alkyl,
$CON(C_1$-$C_3$-Alkyl$)_2$,
$COOC_1$-$C_3$-Alkyl,
COOH,
$NH_2$,
$NHC_1$-$C_3$-Alkyl,
$N(C_1$-$C_3$-Alkyl$)_2$,
NHCHO,
Cl, unter der Voraussetzung, daß $X_3$ und $X_5$ nicht beide Cl sein können,
Br,
I,
F,
$NHCOCH_3$,
$NHCONH_2$,
$NHSO_2CH_3$,
$C_1$-$C_3$-Alkyl COOH,
$NO_2$,
$OC_1$-$C_3$-Alkyl, unter der Voraussetzung, daß $X_4$ nicht $OCH_2CH_3$ sein kann,
$OCOCH_3$,
OH,
$SC_1$-$C_3$-Alkyl,
$SOC_1$-$C_3$-Alkyl,
$SO_2C_1$-$C_3$-Alkyl,
$SO_2NH_2$,
$SO_2NHC_1$-$C_3$-Alkyl,
$SO_2NC(C_1$-$C_3$-Alkyl$)_2$,
$SO_3H$,

und worin die Substituenten an irgendwelchen zwei von $X_2$, $X_3$, $X_4$, $X_5$ oder $X_5$ einen verschmolzenen Ring bilden,
oder worin $R_1$ ausgewählt ist aus der Gruppe bestehend aus wahlweise substituiertem Pyridyl, wahlweise substituiertem Pyrimidyl, 2-Indanyl oder 6-Indazolyl und
worin $R_3$, $R_4$ und $R_5$ Wasserstoff sind und

worin $R_2$ Phenyl ist, das wahlweise durch eine oder zwei Alkoxygruppen oder eine Nitrogruppe, Pyridyl oder 2-Phenylethyl substituiert ist.

**2.** Die Verbindung von Anspruch 1, worin $R_1$ wahlweise substituiertes Phenyl ist, worin $X_4$ ausgewählt ist aus der Gruppe bestehend aus CN, $NO_2$, $CO_2CH_3$, $CONH_2$, HCO, $SO_2NH_2$, $CH_3SO_2$ und $CO_2C_2H_5$.

**3.** Die Verbindung von Anspruch 1, worin $R_1$ ein wahlweise substituiertes Pyridyl ist.

**4.** Die Verbindung von Anspruch 1, worin $R_1$ ein wahlweise substituiertes Pyrimidyl ist.

**5.** Die Verbindung von Anspruch 1, worin $R_2$ ausgewählt ist aus der Gruppe bestehend aus Phenyl, 2-Pyridyl, 3-Pyridyl oder 4-Pyridyl.

**6.** Die Verbindung von Anspruch 1, worin $X_1$ O ist.

**7.** Die Verbindung von Anspruch 1 der Formel:

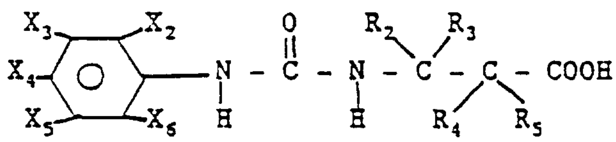

**8.** Die Verbindung von Anspruch 7, worin $X_2$, $X_3$, $X_5$ und $X_5$ H sind und $X_4$ ausgewählt ist aus der Gruppe bestehend aus CH, $NO_2$, $CO_2C_2H_5$, $CO_2CH_3$, $CONH_2$, Cl, Br, F, I, HCO, $CH_3CO$, $SO_2NH_2$ und $CH_3SO_2$.

**9.** Die Verbindung von Anspruch 7, worin $R_2$ ausgewählt ist aus der Gruppe bestehend aus Phenyl, 2-Pyridyl, 3-Pyridyl, 4-Pyridyl.

**10.** Die Verbindung von Anspruch 8, worin $X_4$ ausgewählt ist aus der Gruppe bestehend aus CN, $NO_2$, $CONH_2$, CHO, $CO_2CH_3$ und $CO_2H_2H_5$.

**11.** Die Verbindung von Anspruch 10, worin $R_2$ ausgewählt ist aus der Gruppe bestehend aus 2-Pyridyl, 3-Pyridyl, 4-Pyridyl und Phenyl.

**12.** Die Verbindung von Anspruch 10, worin $X_2$, $X_3$, $X_5$ und $X_5$ H sind, $X_4$ ausgewählt ist aus der Gruppe bestehend aus CN, $NO_2$, $CONH_2$, HCO, $CO_2C_2H_5$, $CO_2CH_3$, Cl, Br, F, I, $CH_3CO$, $CH_2SO_2$ und $SO_2NH_2$, $R_3$, $R_4$ und $R_5$ H sind und $R_2$ ausgewählt ist aus der Gruppe bestehend aus Phenyl, 2-Pyridyl, 3-Pyridyl, 4-Pyridyl.

**13.** Die Verbindung von Anspruch 12, worin $X_4$ CN ist und $R_2$ 3-Pyridyl ist.

**14.** Die Verbindung von Anspruch 12, worin $X_4$ CN ist und $R_2$ Phenyl ist.

**15.** Die Verbindung von Anspruch 12, worin $X_4$ CN ist und $R_2$ 4-Pyridyl ist.

**16.** Die Verbindung von Anspruch 12, worin $X_4$ $NO_2$ ist und $R_2$ Phenyl ist.

**17.** Die Verbindung von Anspruch 12, worin $X_4$ $CO_2C_2H_5$ ist und $R_2$ Phenyl ist.

**18.** Die Verbindung von Anspruch 4, worin $R_3$, $R_4$ und $R_5$ H sind und $R_2$ ausgewählt ist aus der Gruppe bestehend aus Phenyl, 2-Pyridyl, 3-Pyridyl, 4-Pyridyl, Naphthyl.

**19.** Die Verbindung von Anspruch 12, worin $X_4$ $CONH_2$ ist und $R_2$ 3-Pyridyl ist.

**20.** Die Verbindung von Anspruch 13, worin $X_4$ CHO ist und $R_2$ 3-Pyridyl ist.

**21.** Die Verbindung von Anspruch 12, worin $X_4$ $CONH_2$ ist und $R_2$ Phenyl ist.

**22.** Die Verbindung von Anspruch 12, worin $X_4$ CHO ist und $R_2$ Phenyl ist.

**23.** Die Verbindung von Anspruch 12, worin $X_4$ $CONH_2$ ist und $R_2$ 4-Pyridyl ist.

**24.** Die Verbindung von Anspruch 12, worin $X_4$ CHO ist und $R_2$ 4-Pyridyl ist.

**25.** Die Verbindung von Anspruch 19, worin $R_1$ 5-(2-Cyanopyridyl) ist und $R_2$ 3-Pyridyl ist.

**26.** Die Verbindung von Anspruch 19, worin $R_1$ 5-(2-Cyanopyridyl) ist und $R_2$ Phenyl ist.

**27.** Die Verbindung von Anspruch 1, worin die Verbindung ausgewählt ist aus der Gruppe von physiologisch verträglichen Salzen, umfassend Hydrochlorid-, Phosphat-, Citrat-, Sulfat-, Bisulfat-, Natrium-, Kalium, Ammonium-, Calcium-, Malat-, Tosylat-, Benzoat- und Magnesiumsalze.

**28.** Ein Verfahren zum Süßen von eßbaren Produkten, umfassend Nahrungsmittel, Getränke, Konfekt, Kaugummi, Pharmazeutika, Veterinärpräparate und Toiletten-, Kosmetik- und Hygieneprodukte, dadurch gekennzeichnet, daß eine wirksame süßende Menge einer Verbindung von Anspruch 1 diesen eßbaren Produkten zugesetzt wird.

**29.** Eßbare Produkte, gesüßt nach dem Verfahren des Anspruchs 28.

**30.** Süßende Zusammensetzungen, dadurch gekennzeichnet, daß diese Zusammensetzungen eine wirksame süßende Menge einer Verbindung des Anspruchs 1 und einen physiologisch verträglichen Träger dafür enthalten.

**31.** Die süßenden Zusammensetzungen des Anspruchs 30, worin der Träger ein Streckmittel ist.

**32.** Die süßenden Zusammensetzungen des Anspruchs 30, worin der Träger ausgewählt ist aus der Gruppe bestehend aus Wasser, polymerer Dextrose, Stärke und modifizierten Stärken, Maltodextrinen, Cellulose, Methylcellulose, Cellobiitol, Carboxymethylcellulose, Maltit, Hydroxypropylcellulose, Hemicellulosen, mikrokristalliner Cellulose, anderen Cellulosederivaten, Natriumalginat, Pectinen oder anderen Gummis, Lactose, Mallose, Glucose, Leucin, Glycerin, Mannit, Sorbit, Natriumbicarbonat und Phosphor-, Zitronen-, Weinstein-, Fumar-, Benzoe-, Sorbin- und Propionsäuren und deren Natrium-, Kalium- und Calciumsalzen und Gemischen von irgendwelchen der vorstehenden Stoffe.

**33.** Ein süßende Zusammensetzung, enthaltend:
(a) ein erstes Süßemittel, enthaltend eine Verbindung des Anspruchs 1 und
(b) ein zweites Süßemittel, das nicht eine Verbindung des Anspruchs 1 ist.

**34.** Die süßende Zusammensetzung des Anspruchs 33, die außerdem ein Streckmittel enthält.

**35.** Die süßende Zusammensetzung des Anspruchs 33, worin dieses zweite Süßemittel ausgewählt ist aus der Gruppe bestehend aus Saccharose, Maissirupen, Fructose, Aspartam, Alitam, Neohesperidindihydrochalgon, hochfructosehaltigem Maissirup, hydrierter Isomaltulose, Süßemitteln vom steviosiden Typ, L-Zuckern, Lactitol, Neozucker, Glycyrrhizin, Xylit, Acesulfam-K, Natriumsaccharin, Kaliumsaccharin, Calciumsaccharin, Cyclamsäure und Natrium-, Kaum- und Calciumsalzen davon, Sucralose, Monellin, Thaumatin und Gemischen davon.

**36.** Ein Verfahren, umfassend
(a) Umsetzen einer Verbindung der Formel

$R_1NCX_1$

mit einer Verbindung der Formel

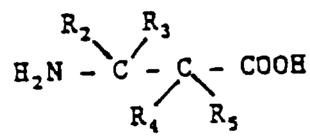

worin $X_1$ O oder S ist, worin die Substituenten $R_1$ -$R_5$ die in Anspruch 1 angegebene Bedeutung haben und

(b) Gewinnen der in vorstehender Stufe (a) gebildeten Hamstoffverbindung.

**37.** Das Verfahren von Anspruch 36, worin $R_1$ ein wahlweise substituiertes Phenyl, wahlweise substituiertes Pyridyl oder wahlweise substituiertes Pyrimidyl ist.

**38.** Das Verfahren des Anspruchs 36, worin $R_1$ ein wahlweise substituiertes Phenyl ist, worin $X_2$, $X_3$, $X_5$ und $X_5$ H sind und $X_4$ ausgewählt ist aus der Gruppe bestehend aus CN, $NO_2$, $CO_2C_2H_5$, $CO_2CH_3$, $CONH_2$, Cl, Br, F, I, HCO, $CH_3CO$, $SO_2NH_2$ und $CH_3SO_3$ und $X_1$ O ist.

**39.** Das Verfahren des Anspruchs 36, worin $R_2$ ausgewählt ist aus der Gruppe bestehend aus Phenyl, 2-Pyridyl, 3-Pyridyl, 4-Pyridyl.

**40.** Das Verfahren des Anspruchs 36, worin $X_4$ ausgewählt ist aus der Gruppe bestehend aus CN, $NO_2$, $CONH_2$, CHO, $CH_2CH_3$ und $CO_2C_2H_5$.

**41.** Das Verfahren des Anspruchs 36, worin $R_2$ ausgewählt ist aus der Gruppe bestehend aus 2-Pyridyl, 3-Pyridyl, 4-Pyridyl und Phenyl.

**42.** Das Verfahren des Anspruchs 36, worin $R_1$ ein wahlweise substituiertes Pyridyl ist.

**43.** Das Verfahren des Anspruchs 36, worin $R_1$ ein wahlweise substituiertes Pyrimidyl ist.

**44.** Das Verfahren des Anspruchs 36, worin Stufe (a) in Gegenwart einer Base durchgeführt wird.

**45.** Das Verfahren des Anspruchs 36, worin Stufe (a) in Gegenwart eines Lösungsmittels durchgeführt wird.

**46.** Das Verfahren des Anspruchs 45, worin dieses Lösungsmittel Acetonitril ist.

**47.** Das Verfahren des Anspruchs 45, worin dieses Lösungsmittel ein Gemisch aus Acetonitril und Wasser ist.

**48.** Ein süßes Nahrungsmittel, enthaltend eine oder mehrere Verbindungen des Anspruchs 1 als Süßemittel.

**49.** Eine eßbare Zusammensetzung, enthaltend

(a) ein Nahrungsmittel und

(b) ein oder mehrere Süßemittel, ausgewählt aus der Gruppe bestehend aus den Verbindungen des Anspruchs 1.

**50.** Ein Verfahren, umfassend

(a) Umsetzen einer Verbindung der Formel

$R_1NH_2$

mit einer Verbindung der Formel

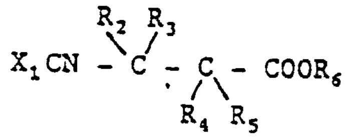

worin $X_1$ O oder S ist, worin die Substituenten $R_1$-$R_5$ die in Anspruch 1 angegebene Bedeutung besitzen und worin $R_6$ Methyl, Ethyl, Propyl oder Butyl ist und
(b) Hydrolysieren der dabei entstehenden Verbindung und
(c) Gewinnen der in Stufe (a) gebildeten isolierten gewünschten Hamstoffverbindung oder eines Salzes davon.

**51.** Die eßbare Zusammensetzung des Anspruchs 49, die außerdem ein Süßemittel enthält, ausgewählt aus der Gruppe bestehend aus Saccharose, Maissirupen, Fructose, Aspartam, Alitam, Neohesperidin-dihydrochalgon, hochfructosehaltigem Maissirup, hydrierter Isomaltulose, Süßemitteln vom Steviosid-Typ, L-Zuckem, Lactitol, Neozucker, Glycyrrhizin, Xylit, Acesulfam-K, Natriumsaccharin, Kaliumsaccharin, Calciumsaccharin, Cyclamsäure und den Natrium-, Kalium- und Calciumsalzen davon, Sucralose, Monellin, Thaumatin und Gemischen davon.

**52.** Die eßbare Zusammensetzung des Anspruchs 49, umfassend ein Getränk.

**53.** Die eßbare Zusammensetzung des Anspruchs 49, umfassend ein Konfekt.

**54.** Eine Verbindung zur Verwendung für die Herstellung der Zusammensetzungen des Anspruchs 1, entsprechend der Formel

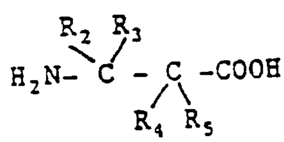

oder deren Salze, worin $R_2$, $R_3$, $R_4$ und $R_5$ die in Anspruch 1 angegebene Bedeutung haben.

**55.** Ein Verfahren zur Herstellung eines ersten Harnstoffs oder Thioharnstoffs der Formel

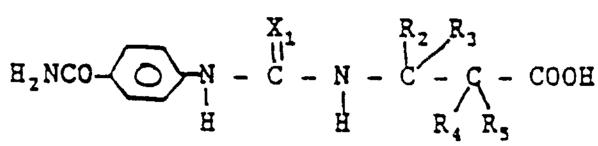

und allen Salzen davon,
eines zweiten Harnstoffs oder Thioharnstoffs der Formel

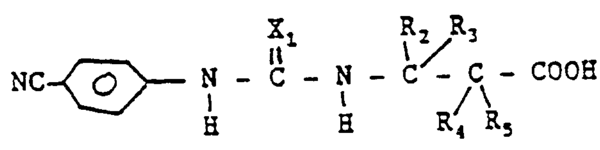

und allen Salzen davon,
worin $X_1$ O oder S ist und worin
$R_2$, $R_3$, $R_4$ und $R_5$ die in Anspruch 1 angegebene Bedeutung besitzen, umfassend Enantiomere und physiologisch verträgliche Salze davon, wobei dieses Verfahren folgende Stufen umfaßt:
Umsetzung dieses zweiten Harnstoffs oder Thioharnstoffs mit Alkaliwasserstoffperoxid unter Bildung

des ersten Harnstoffs oder Thioharnstoffs.

**56.** Ein Verfahren zur Herstellung von einem Isomeren einer ersten Verbindung der Formel

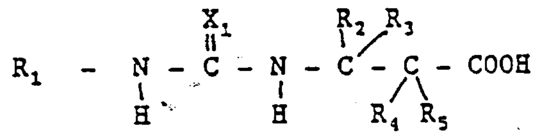

und allen Salzen davon, worin $X_1$ O oder S ist und $R_1$ -$R_5$ die in Anspruch 1 angegebene Bedeutung besitzen,
umfassend folgende Stufen:
Umsetzung eines Aldehyds mit einem Amin unter Bildung einer Schiff'schen Base;
Umsetzung dieser Schiff'schen Base mit einem Methylhalogenacetat und einem Metall unter Bildung eines diastereomeren Gemischs eines β-Lactams;
Hydrolysieren dieses β-Lactams unter Bildung eines diastereomeren Gemischs einer ersten β-Aminosäure;
Veresterung dieser ersten β-Aminosäure;
Isolieren eines Isomers des Esters dieses diastereomeren Gemischs dieser ersten β-Aminosäure;
Hydrolysieren dieses Esters unter Bildung eines Stereoisomeren einer zweiten β-Aminosäure;
Umsetzung dieses Stereoisomeren der zweiten Aminosäure mit einem Isocyanat oder Isothiocyanat unter Bildung dieser ersten Verbindung.

**57.** Das Verfahren des Anspruchs 56, worin dieses Metall Zink ist.

**58.** Das Verfahren des Anspruchs 56, worin diese zweite β-Aminosäure hergestellt worden ist durch Umsetzung der ersten β-Aminosäure mit Palladium und Kohle.

**59.** Ein Verfahren zur Herstellung eines Isomeren einer ersten Verbindung der Formel

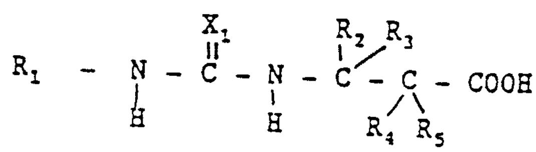

und allen Salzen davon, worin $X_1$ O oder S ist, $R_1$-$R_5$ die in Anspruch 1 angegebene Bedeutung haben,
umfassend folgende Stufen:
Umsetzen eines Aldehyds mit einem Amin unter Bildung einer Schiff'schen Base;
Umsetzen dieser Schif'schen Base mit Methylhalogenacetat und einem Metall unter Bildung eines diastereomeren Gemischs eines β-Lactams;
Isolieren eines Diastereomers dieses β-Lactams;
Hydrolysieren dieses Isomers dieses β-Lactams unter Bildung eines Stereoisomeren der ersten β-Aminosäure;
Hydrolysieren dieses Stereoisomers der ersten β-Aminosäure unter Bildung eines Stereoisomeres dieser zweiten β-Aminosäure;
Umsetzen dieses Stereoisomers dieser zweiten β-Aminosäure mit einem Isocyanat oder Isothiocyanat unter Bildung dieser ersten Verbindung.

**Revendications**

**1.** Composé répondant à la formule

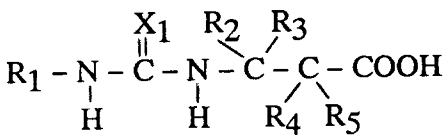

dans laquelle $X_1$ est O ou S, dans laquelle $R_1$ est un groupement phényle facultativement substitué, ledit phényle répondant à la formule

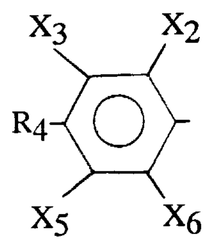

dans laquelle $X_2$, $X_3$, $X_4$, $X_5$ et $X_5$ sont identiques ou différents et sont sélectionnés dans le groupe constitué par :

H,
$CF_3$,
$CF_2CF_3$,
$CH_2CF_3$,
alkyle en $C_1$-$C_4$,
$CH=NOCH_3$,
$CH=NOH$,
CHO,
$CH_2OCH_3$,
$CH_2OH$,
CN,
$COCF_3$,
CO-alkyle en $C_1$-$C_3$,
$CONH_2$,
CONH-alkyle en $C_1$-$C_3$,
CON(alkyle en $C_1$-$C_3$)$_2$,
COO-alkyle en $C_1$-$C_3$,
COOH,
$NH_2$,
NH-alkyle en $C_1$-$C_3$,
N(alkyle en $C_1$-$C_3$)$_2$,
NHCHO,
Cl, à la condition que $X_3$ et $X_5$ ne puissent être l'un et l'autre Cl,
Br,
I,
F,
$NHCOCH_3$,
$NHCONH_2$,
$NHSO_2CH_3$,
COOH-alkyle en $C_1$-$C_3$,
$NO_2$,
O-alkyle en $C_1$-$C_3$, à la condition que $X_4$ ne puisse être $OCH_2CH_3$,

$OCOCH_3$,
OH,
S-alkyle en $C_1$-$C_3$,
SO-alkyle en $C_1$-$C_3$,
$SO_2$-alkyle en $C_1$-$C_3$,
$SO_2NH_2$,
$SO_2NH$-alkyle en $C_1$-$C_3$,
$SO_2NC$(alkyle en $C_1$-$C_3$)$_2$,
$SO_3H$,

et dans laquelle des substituants en deux positions quelconques parmi $X_2$, $X_3$, $X_4$, $X_5$ ou $X_5$ forment une combinaison cyclique condensée,
ou dans laquelle $R_1$ est sélectionné dans le groupe constitué par un groupement pyridyle facultativement substitué, un groupement pyrimidyle facultativement substitué, un groupement 2-indanyle ou un groupement 6-indazolyle et
dans laquelle $R_3$, $R_4$ et $R_5$ sont un atome d'hydrogène et
dans laquelle $R_2$ est un groupement phényle facultativement substitué par un ou deux groupements alcoxy ou un groupement nitro, un groupement pyridyle ou un groupement 2-phényléthyle.

**2.** Composé selon la revendication 1, dans lequel $R_1$ est un groupement phényle facultativement substitué dans lequel $X_4$ est sélectionné dans le groupe constitué par CN, $NO_2$, $CO_2CH_3$, $CONH_2$, HCO, $SO_2NH_2$, HCO, $CH_3SO_2$ et $CO_2C_2H_5$.

**3.** Composé selon la revendication 1, dans lequel $R_1$ est un groupement pyridyle facultativement substitué.

**4.** Composé selon la revendication 1, dans lequel $R_1$ est un groupement pyrimidyle facultativement substitué.

**5.** Composé selon la revendication 1, dans lequel $R_2$ est sélectionné dans le groupe constitué par les groupements phényle, 2-pyridyle, 3-pyridyle ou 4-pyridyle.

**6.** Composé selon la revendication 1, dans lequel $X_1$ est 0.

**7.** Composé selon la revendication 1, de formule :

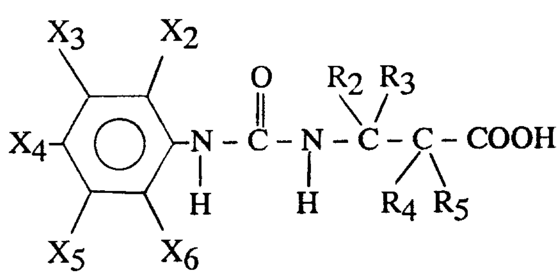

**8.** Composé selon la revendication 7, dans lequel $X_2$, $X_3$, $X_5$ et $X_5$ sont H et $X_4$ est sélectionné dans le groupe constitué par CN, $NO_2$, $CO_2C_2H_5$, $CO_2CH_3$, $CONH_2$, Cl, Br, F, I, HCO, $CH_3CO$, $SO_2NH_2$ et $CH_3SO_2$.

**9.** Composé selon la revendication 7, dans lequel $R_2$ est sélectionné dans le groupe constitué par les groupements phényle, 2-pyridyle, 3-pyridyle, 4-pyridyle.

**10.** Composé selon la revendication 8, dans lequel $X_4$ est sélectionné dans le groupe constitué par CN, $NO_2$, $CONH_2$, CHO, $CO_2CH_3$ et $CO_2C_2H_5$.

**11.** Composé selon la revendication 10, dans lequel $R_2$ est sélectionné dans le groupe constitué par les groupements 2-pyridyle, 3-pyridyle, 4-pyridyle et phényle.

**12.** Composé selon la revendication 10, dans lequel $X_2$, $X_3$, $X_5$ et $X_5$ sont H, $X_4$ est sélectionné dans le groupe constitué par CH, $NO_2$, $CONH_2$, HCO, $CO_2C_2H_5$, $CO_2CH_3$, Cl, Br, F, I, $CH_3CO$, $CH_3SO_2$ et $SO_2NH_2$, $R_3$, $R_4$ et $R_5$ sont H, et $R_2$ est sélectionné dans le groupe constitué par les groupements phényle, 2-pyridyle, 3-pyridyle, 4-pyridyle.

**13.** Composé selon la revendication 12, dans lequel $X_4$ est CN et $R_2$ est un groupement 3-pyridyle.

**14.** Composé selon la revendication 12, dans lequel $X_4$ est CN et $R_2$ est un groupement phényle.

**15.** Composé selon la revendication 12, dans lequel $X_4$ est CN et $R_2$ est un groupement 4-pyridyle.

**16.** Composé selon la revendication 12, dans lequel $X_4$ est $NO_2$ et $R_2$ est un groupement phényle.

**17.** Composé selon la revendication 12, dans lequel $X_4$ est $CO_2C_2H_5$ et $R_2$ est un groupement phényle.

**18.** Composé selon la revendication 4, dis lequel $R_3$, $R_4$ et $R_5$ sont H et $R_2$ est sélectionné dans le groupe constitué par les groupements phényle, 2-pyridyle, 3-pyridyle, 4-pyridyle ou naphtyle.

**19.** Composé selon la revendication 12, dans lequel $X_4$ est $CONH_2$ et $R_2$ est un groupement 3-pyridyle.

**20.** Composé selon la revendication 13, dans lequel $X_4$ est CHO et $R_2$ est un groupement 3-pyridyle.

**21.** Composé selon la revendication 12, dans lequel $X_4$ est $CONH_2$ et $R_2$ est un groupement phényle.

**22.** Composé selon la revendication 12, dans lequel $X_4$ est CHO et $R_2$ est un groupement phényle.

**23.** Composé selon la revendication 12, dis lequel $X_4$ est $CONH_2$ et $R_2$ est un groupement 4-pyridyle.

**24.** Composé selon la revendication 12, dans lequel $X_4$ est CHO et $R_2$ est un groupement 4-pyridyle.

**25.** Composé selon la revendication 19, dans lequel $R_1$ est un groupement 5-(2-cyanopyridyle) et $R_2$ un groupement 3-pyridyle.

**26.** Composé selon la revendication 19, dans lequel $R_1$ est un groupement 5-(2-cyanopyridyle) et $R_2$ est un groupement phényle.

**27.** Composé selon la revendication 1, dans lequel le composé est sélectionné dans le groupe de sels physiologiquement acceptables comprenant chlorhydrate, phosphate, citrate, sulfate, bisulfate, sodium, potassium, ammonium, calcium, malate, tosylate, benzoate et sels de magnésium.

**28.** Procédé d'édulcoration de produits comestibles comprenant produits alimentaires, boissons, confiseries, gommes à mâcher, produits pharmaceutiques, préparations vétérinaires, et articles de toilette, articles cosmétiques et d'hygiène, caractérisé en ce qu'une quantité édulcorante efficace d'un composé selon la revendication 1 est ajoutée auxdits produits comestibles.

**29.** Produits comestibles édulcorés selon le procédé de la revendication 28.

**30.** Compositions édulcorantes, caractérisées en ce que lesdites compositions comprennent une quantité édulcorante efficace d'un composé selon la revendication 1 et un véhicule physiologiquement acceptable destiné à ce dernier.

**31.** Compositions édulcorantes selon la revendication 30, dans lesquelles le véhicule est un diluant.

**32.** Compositions édulcorantes selon la revendication 30, dans lesquelles le véhicule est sélectionné dans le groupe constitué par l'eau, le dextrose polymère, l'amidon et les amidons modifiés, les maldodextrines, la cellulose, la méthylcellulose, le cellobitol, la carboxyméthylcellulose, le maltitol, l'hydroxypropylcellulose, les hémicelluloses, la cellulose microcristalline, d'autres dérivés cellulosiques, l'alginate de sodium, les pectines et autres gommes, le lactose, le maltose, le glucose, la leucine, le glycérol, le mannitol, le sorbitol, le bicarbonate de sodium et les acides phosphorique, citrique, tartrique, fumarique, benzoïque, sorbique et propionique et leurs sels de sodium, potassium et calcium et des mélanges des substances susmentionnées.

**33.** Composition édulcorante comprenant :
(a) un premier agent édulcorant comprenant un composé selon la revendication 1 et
(b) un second agent édulcorant qui n'est pas un composé selon la revendication 1.

**34.** Composition édulcorante selon la revendication 33, comprenant en outre un diluant.

**35.** Composition édulcorante selon la revendication 33, dans laquelle ledit second agent édulcorant est sélectionné dans le groupe constitué par le saccharose, les sirops de maïs, le fructose, l'aspartame, l'alitame, la dihydrochalcone de néohespéridine, le sirop de maïs à teneur élevée en fructose, l'isomaltulose hydrogéné, les édulcorants de type stévioside, les L-sucres, le lactitol, le néosucre, la glycyrrhizine, le xylitol, l'acésulfame-K, la saccharine de sodium, la saccharine de potassium, la saccharine de calcium, l'acide cyclamique et ses sels de sodium, potassium et calcium, le sucralose, la monelline, la thaumatine et leurs mélanges.

**36.** Procédé comprenant :
(a) la mise à réagir d'un composé de formule :

$R_1NCX_1$

avec un composé de formule

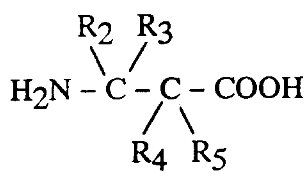

dans laquelle $X_1$ est O ou S, dans laquelle les substituants $R_1$ à $R_5$ sont définis selon la revendication 1 ; et
(b) la récupération du composé durée formé à l'étape (a) ci-dessus.

**37.** Procédé selon la revendication 36, dans lequel $R_1$ est un groupement phényle facultativement substitué, un groupement pyridyle facultativement substitué ou un groupement pyrimidyle facultativement substitué.

**38.** Procédé selon la revendication 36, dans lequel $R_1$ est un groupement phényle facultativement substitué dans lequel $X_2$, $X_3$, $X_5$ et $X_5$ sont H et $X_4$ est sélectionné dans le groupe constitué par CN, $NO_2$, $CO_2C_2H_5$, $CO_2CH_3$, $CONH_2$, Cl, Br, F, I, HCO, $CH_3CO$, $SO_2NH_2$ et $CH_3SO_2$, et $X_1$ est O.

**39.** Procédé selon la revendication 36, dans lequel $R_2$ est sélectionné dans le groupe constitué par les groupements phényle, 2-pyridyle, 3-pyridyle, 4-pyridyle.

**40.** Procédé selon la revendication 36, dans lequel $X_4$ est sélectionné dans le groupe constitué par CN, $NO_2$, $CONH_2$, CHO, $CO_2CH_3$ et $CO_2C_2H_5$.

**41.** Procédé selon la revendication 36, dans lequel $R_2$ est sélectionné dans le groupe constitué par les groupements 2-pyridyle, 3-pyridyle, 4-pyridyle et phényle.

**42.** Procédé selon la revendication 36, dans lequel $R_1$ est un groupement pyridyle facultativement substitué.

**43.** Procédé selon la revendication 36, dans lequel $R_1$ est un groupement pyrimidyle facultativement substitué.

**44.** Procédé selon la revendication 36, dans lequel l'étape (a) est mise en oeuvre en présence d'une base.

**45.** Procédé selon la revendication 36, dans lequel l'étape (a) est mise en oeuvre en présence d'un solvant.

**46.** Procédé selon la revendication 45, dans lequel ledit solvant est de l'acétonitrile.

**47.** Procédé selon la revendication 45, dans lequel ledit solvant est un mélange d'acétonitrile et d'eau.

**48.** Denrée alimentaire sucrée comprenant un ou plusieurs composés selon la revendication 1 comme agent édulcorant.

**49.** Composition comestible comprenant
(a) une denrée alimentaire ; et
(b) un ou plusieurs agents édulcorants sélectionnés dans le groupe constitué par les composés selon la revendication 1.

**50.** Procédé comprenant
(a) la mise à réagir d'un composé de formule :

$R_1NH_2$

avec un composé de formule

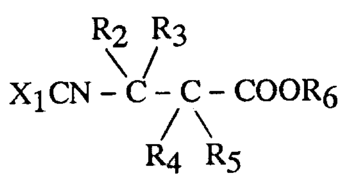

dans laquelle $X_1$ est O ou S, dans laquelle les substituants $R_1$ à $R_5$ sont définis selon la revendication 1 et dans laquelle $R_6$ est un groupement méthyle, éthyle, propyle ou butyle, et
(b) l'hydrolyse du composé obtenu ; et
(c) la récupération du composé d'urée désiré isolé ou de son sel, formé à l'étape (a).

**51.** Composition comestible selon la revendication 49, comprenant en outre un agent édulcorant sélectionné dans le groupe constitué par le saccharose, les sirops de maïs, le fructose, l'aspartame, l'alitame, la dihydrochalcone de néohespéridine, le sirop de maïs à teneur élevée en fructose, l'isomaltulose hydrogéné, les édulcorants de type stévioside, les L-sucres, le lactitol, le néosucre, la glycyrrhizine, le xylitol, l'acésulfame-K la saccharine de sodium, la saccharine de potassium, la saccharine de calcium, l'acide cyclamique et ses sels de sodium, potassium et calcium, le sucralose, la monelline, la thaumatine et leurs mélanges.

**52.** Composition comestible selon la revendication 49, comprenant une boisson.

**53.** Composition comestible selon la revendication 49, comprenant une confiserie.

**54.** Composé à utiliser dans la préparation des compositions selon la revendication 1, répondant à la formule

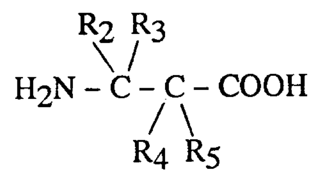

ou ses sels
dans laquelle $R_2$, $R_3$, $R_4$ et $R_5$ sont définis selon la revendication 1.

**55.** Procédé de production d'une première urée ou thiourée de formule

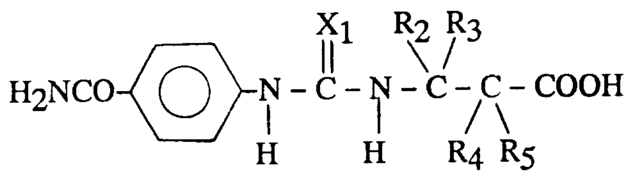

et tous ses sels
à partir d'une seconde urée ou thiourée de formule

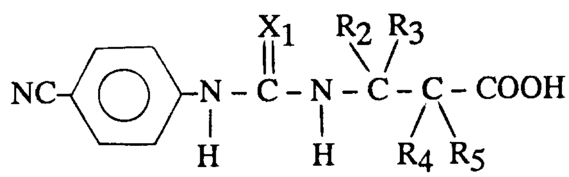

et tous ses sels
dans laquelle $X_1$ est O ou S et dans laquelle
$R_2$, $R_3$, $R_4$ et $R_5$ sont définis selon la revendication 1, comprenant énantiomères et sels physiologiquement acceptables, ledit procédé comprenant l'étape consistant à : mettre à réagir ladite seconde urée ou thiourée avec du peroxyde d'hydrogène alcalin pour produire ladite première urée ou thiourée.

**56.** Procédé d'obtention d'un isomère d'un premier composé de formule :

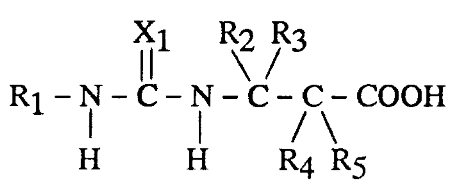

et tous ses sels
dans laquelle $X_1$ est O ou S, $R_1$ à $R_5$ sont définis selon la revendication 1, comprenant les étapes consistant à :
mettre à réagir un aldéhyde avec une amine pour produire une base de Schiff;
mettre a réagir ladite base de Schiff avec un haloacétate de méthyle et un métal pour produire un mélange diastéréoisomère d'une $\beta$-lactame;
hydrolyser ladite $\beta$-lactame pour produire un mélange diastéréoisomère d'un premier $\beta$-aminoacide ;
estérifier ledit premier $\beta$-aminoacide ;
isoler un isomère de l'ester dudit mélange diastéréoisomère dudit premier $\beta$-aminoacide;
hydrogénolyser ledit ester pour produire un stéréoisomère d'un second $\beta$-aminoacide ;

mettre à réagir ledit stéréoisomère du second aminoacide avec un isocyanate ou un isothiocyanate pour produire ledit premier composé.

**57.** Procédé selon la revendication 56, dans lequel ledit métal est du zinc.

**58.** Procédé selon la revendication 56, dans lequel ledit second $\beta$-aminoacide est produit par réaction entre ledit premier $\beta$-aminoacide et du palladium et du carbone.

**59.** Procédé d'obtention d'un isomère d'un premier composé de formule :

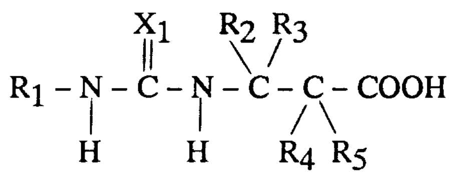

et tous ses sels
dans laquelle $X_1$ et O ou S, $R_1$ à $R_5$ sont définis selon la revendication 1, comprenant les étapes consistant à :
mettre à réagir un aldéhyde avec une amine pour produire une base de Schiff ;
mettre à réagir ladite base de Schiff avec un haloacétate de méthyle et un métal pour produire un mélange diastéréoisomère d'une $\beta$-lactame ;
isoler un diastéréoisomère de ladite $\beta$-lactame ;
hydrolyser ledit isomère de ladite $\beta$-lactame pour produire un stéréoisomère d'un premier $\beta$-aminoacide ;
hydrogénolyser ledit stéréoisomère du premier $\beta$-aminoacide pour produire un stéréoisomère dudit second $\beta$-aminoacide ;
mettre à réagir ledit stéréoisomère dudit second $\beta$-aminoacide avec un isocyanate ou un isothiocyanate pour produire ledit premier composé.